# EUROPEAN PATENT SPECIFICATION

(11) **EP 2 596 702 B1**
(45) Date of publication and mention of the grant of the patent: **27.05.2015**
(21) Application number: 11190704.4
(22) Date of filing: 25.11.2011
(51) Int. Cl.: A01N 33/04, A01N 35/02, A01N 43/80, A01P 1/00, A01N 31/08, C09C 1/02, D21H 21/36

(54) **Process for stabilizing bacterial content of aqueous ground natural calcium carbonate and/or precipitated calcium carbonate and/or dolomite and/or surface-reacted calcium carbonate-comprising mineral preparations**
Verfahren zur Bekämpfung von Bakterien in wässrigen Dispersionen von gemahlenem natürlichen Calciumcarbonat und/oder ausgefälltem Calciumcarbonat und/oder Dolomit und/oder oberflächebehandelten calciumcarbonathaltigen Mineralpräparaten
Procédé pour la stabilisation bactérienne du carbonate de calcium aqueux naturel et/ou du carbonate de calcium précipité et/ou de la dolomite et/ou de préparations minérales comprenant du carbonate de calcium à réaction en surface

(43) Date of publication of application: 29.05.2013
(73) Proprietor: Omya International AG, 4665 Oftringen (CH)
(72) Inventor: Di Maiuta, Nicola, 4528 Zuchwil (CH); Schwarzentruber, Patrick, 5245 Habsburg (CH)
(74) Representative: Tiefbrunner, Vera

(56) References cited:
- EP-A1- 2 329 712
- WO-A1-2007/042214
- DE-A1-102009 048 188

## Description

The invention relates to a process for stabilizing bacterial content of aqueous mineral preparations comprising at least one of ground natural calcium carbonate, precipitated calcium carbonate, dolomite, surface-reacted calcium carbonate, clay, talc, TiO₂, kaolin, kaolinitic clay, calcined kaolinitic clay, calcium sulfate, quartz, attapulgite, montmorillonite, diatomaceous earth, finely divided silica, aluminium oxide, aluminium hydroxide, silicates such as aluminium silicate, pumice and sepiolite, or a mixture thereof, and to the use of biocidal activity enhancing compounds.

In practice, aqueous preparations and especially suspensions or dispersions of water-insoluble mineral solids are used extensively in the paper, paint, rubber and plastics industries as coatings, fillers, extenders and pigments for papermaking as well as aqueous lacquers and paints. For example, suspensions or dispersions of mineral solids are used in the paper industry in large amounts as filler and/or as a component in the preparation of coated paper. Typical aqueous preparations of water-insoluble solids are characterized in that they comprise water, a water-insoluble solid compound and optionally further additives, such as dispersing agents, in the form of a suspension or dispersion. Water-soluble polymers and copolymers which may be used as e.g. dispersant and/or grinding aid in such preparation are, for example, described in US 5,278,248.

The aforementioned aqueous preparations are often subject to contamination by microorganisms such as aerobic and anaerobic bacteria resulting in changes in the preparation properties, such as discolorations or reductions in other quality parameters, which negatively affect their commercial value. Therefore, the manufacturers of such aqueous preparations usually take measures for stabilizing the suspensions, dispersions or slurries. For example, it is known that aldehyde-releasing biocides reduce the growth and accumulation of such microorganisms in aqueous preparations and, thus, reduce the tendency of undesired alterations of these preparations, like unpleasant odours.

For ensuring an acceptable microbiological quality of aqueous preparations, biocides are used over the entire life cycle of the preparation (production, storage, transport, use). In the art, several approaches for improving the microbiological quality of aqueous preparations have been proposed. For example, EP 1 139 741 describes aqueous suspensions or dispersions of minerals, fillers and/or pigments, containing a microbicidal agent in the form of a solution and derivatives of phenol in partially neutralized form. US 5,496,398 relates to a process for the reduction of microorganisms in kaolin clay slurries by a combination of low temperature heat and reduced levels of a microbiocidal agent. WO 02/052941 describes biocide compositions for incorporation into paints, coating, plasters and plastics comprising at least one metal oxide and at least one metal salt. US 2006/0111410 mentions a mixture comprising 1,2-benzisothiazolinone (BIT) and tetramethylolacetylenediurea (TMAD) for protecting industrial materials and products against attack and destruction by microorganisms. Furthermore, it is suggested in the art to add formaldehyde-releasing substances to such aqueous preparations for improving the microbiologically related quality. For example, US 4,655,815 mentions an antimicrobial composition comprising a formaldehyde donor. Furthermore, WO 2006/079911 describes a method of protection against microorganisms by increasing the OH- ion concentration of the suspension.

WO 2004/040979 A1 relates to synergistic antimicrobial mixtures containing 1,2-benzisothiazolinon (BIT) and benzylhemiformal (BHF). The corresponding mixtures are used, for example, for suspensions of pigments.

EP 1 661 587 A1 relates to germicidal compositions including phthalaldehyde as an active ingredient. It is indicated in EP 1 661 587 A1 that carbonate salts and bicarbonate salts may enhance the germicidal efficacy of phthalaldehydes.

US 2001/0009682 A1 relates to disinfectant concentrates having improved biocidal activity which may contain an aldehyde such as glutaraldehyde, a glycol and a lithium based buffer.

EP 2 108 260 refers to a process for bacterial stabilizing of an aqueous preparation, said preparation comprising at least one mineral and at least one strain of bacteria which is resistant to, tolerant to and/or degrade aldehyde-releasing and/or aldehyde-based biocides, wherein the process comprises the steps of: (a) adding to the aqueous preparation one or more aldehyde-releasing and/or aldehyde-based biocides in an amount such that the total amount of aldehyde-releasing and/or aldehyde-based biocides in the aqueous preparation is from 250 ppm to 5 000 ppm, calculated relative to the water in the preparation; (b) adding at least one water soluble lithium compound to the aqueous preparation in an amount such that the total amount of solubilised lithium in the aqueous preparation is from 1 000 to 3 000 ppm, calculated relative to the weight of water in the preparation, where steps (a) and (b) may be carried out simultaneously, or separately in any order.

Finally, EP 2329 712 discloses a process for stabilising an aqueous mineral preparation comprising a step of: (a) adding at least one aldehyde-containing and/or aldehyde-releasing and/or phenolic and/or isothiazoline biocide to said aqueous mineral preparation; wherein said mineral comprises at least one of: a ground natural calcium carbonate, a precipitated calcium carbonate, a dolomite, a surface-reacted calcium carbonate, or a mixture thereof; said process comprises a step (b), which may be simultaneous and/or distinct relative to step (a), of adding at least one monoalcohol primary alkanol amine to said aqueous mineral preparation; said biocide(s) are added to said aqueous preparation in an amount corresponding to from 90 to 1 350 ppm based on the weight of the aqueous phase of said aqueous preparation; and wherein said monoalcohol primary alkanol amine(s) are added to said aqueous preparation in an amount corresponding to from 600 to 1 200 ppm based on the weight of the aqueous phase of said aqueous preparation.

DE 10 2009 048 188 relates to antimicrobially effective use solutions and the use of combinations of isothiazolinones and amines for preserving use solutions.

WO2007/042214 A1 relates to biocidal active mixtures comprising o-phenylphenol and amines and their use for protecting industrial materials.

Because of the limited activity spectrum of several biocides, the efficacy of such biocides against bacteria is not always satisfactory and, thus, the obtained action is in some cases insufficient to avoid microbially induced alteration of aqueous preparations.

Thus, there is still a need for adequate compositions providing sufficient biocidal activity in aqueous preparations such as suspensions and dispersions of mineral materials comprising ground natural calcium carbonate in order to achieve a longer lasting and sufficient stabilization.

These and other objectives of the present invention can be solved by a process and a use as described in the present invention and defined in the claims.

One aspect of the present application resides in a process for stabilising an aqueous mineral preparation comprising a step of:
(a) adding at least one aldehyde-containing and/or aldehyde-releasing and/or phenolic and/or isothiazoline biocide to said aqueous mineral preparation;
   characterised in that:
   - said mineral comprises at least one of: a ground natural calcium carbonate, a precipitated calcium carbonate, a dolomite, a surface-reacted calcium carbonate, clay, talc, TiO₂, kaolin, kaolinitic clay, calcined kaolinitic clay, calcium sulfate, quartz, attapulgite, montmorillonite, diatomaceous earth, finely divided silica, aluminium oxide, aluminium hydroxide, silicates such as aluminium silicate, pumice and sepiolite, or a mixture thereof;
   - said process comprises a step (b), which may be simultaneous and/or distinct relative to step (a), of adding at least one linear or cyclic diamine or triamine compound to said aqueous mineral preparation, the linear or cyclic diamine or triamine compound having at least one primary amine group, and where the linear diamine or triamine compound has the following formula (I): 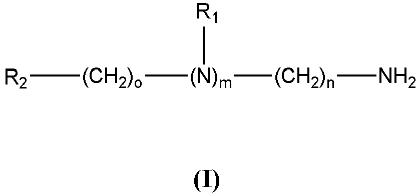 wherein:
      m is an integer and is either 0 or 1, n is an integer and is from 2 to 8, o is an integer and is from 0 to 3, R₁ is a C₁ to C₁₂ alkyl group, and is in particular CH₃, CH₂CH₃ or
      a linear C₁₂H₂₅ alkyl group, and R₂ is CH₃ or NH₂, and with the proviso that when m = 0, then o = 0 and R₂ is NH₂;
      and where the cyclic diamine or triamine compound has one of the following formulas (II) and (III): 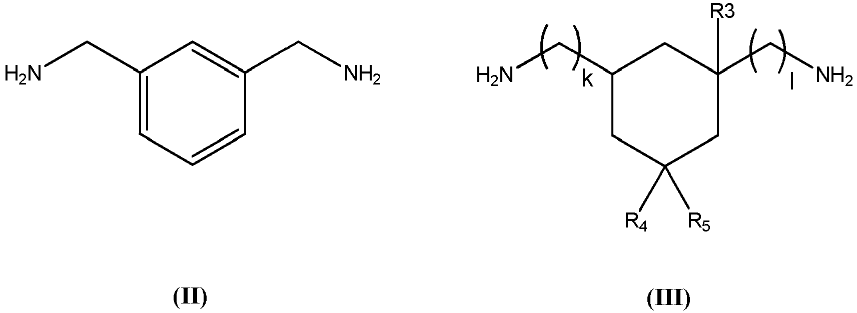
      wherein:
      k and 1 are the same or different and are either 0 or 1, R₃ is either H or CH₃, and R₄ and R₅ are the same or different and selected from H and CH₃;
   - said biocide(s) are added to said aqueous preparation in an amount corresponding to from 90 to 1 350 ppm, based on the weight of the aqueous phase of said aqueous preparation; and
   - said linear or cyclic diamine or triamine compound(s) are added to said aqueous preparation in an amount corresponding to from 600 to 1 200 ppm, based on the weight of the aqueous phase of said aqueous preparation.

According to the present invention, the wording "stabilising an aqueous preparation" implies no "significant growth" of bacteria. Preferably, the stabilization leads to a reduction and/or maintenance of the Total Viable Count (TVC, given in colony forming units per milliliter (cfu/ml), as measured according to the measurement method defined in the Examples section hereafter) of the treated aqueous preparation to a value of less than 10⁴ cfu/ml, more preferably to a value of less than 10³ cfu/ml, and even more preferably to a value of less than or equal to 10² cfu/ml.

An "aqueous mineral preparation" in the meaning of the present invention is a suspension comprising ground natural calcium carbonate and/or precipitated calcium carbonate and/or dolomite and/or surface-reacted calcium carbonate-comprising minerals and water and optionally further additives. Preparations having the required solids content may be viscous and require the implementation of dispersing agents or other rheology modifying agents.

The solids content in the meaning of the present application corresponds to the residual weight of the aqueous preparation following evaporation of the aqueous phase and is determined according to the measurement method described in the Examples section herebelow.

The weight of the aqueous phase is determined by subtracting the residual weight of the aqueous preparation following evaporation of the aqueous phase (determined according to the measurement method described in the Examples section herebelow) from the total weight of the aqueous preparation.

In accordance with the present invention, an "aldehyde-releasing biocide" refers to a compound which is able to release mono- di-, and/or tri-aldehyde. Aldehyde-releasing biocides include, for example, (ethylenedioxy)dimethanol, which releases formaldehyde.

In accordance with the present invention, an "aldehyde-based biocide" refers to a biocide which has one or more aldehyde-groups. Aldehyde-based biocides include, for example, formaldehyde, acetaldehyde, propionaldehyde, glutardialdehyde and glyoxal.

In accordance with the present invention, a "phenolic biocide" refers to a biocide which comprises at least one phenol functional group.

In accordance with the present invention, an "isothiazoline biocide" refers to a biocide which comprises at least one isothiazoline group.

According to the present invention, the content of the aldehyde-releasing and/or aldehyde-based and/or phenolic and/or isothiazoline biocides in water can be evaluated by HPLC (high pressure liquid chromatography). If necessary, the corresponding aldehyde-releasing and/or aldehyde-based and/or phenolic and/or isothiazoline biocide may be converted into a derivative before evaluation with HPLC.

The term "mineral" in the meaning of the present invention encompasses not only natural occurring pigments, fillers and minerals, such as marble, talc, chalk, dolomite, limestone and the like, but also synthetic compounds that are man-made and that show the same or similar properties, for example in an X-ray diffraction spectrum, like naturally occurring pigments, fillers and minerals. One example for such a synthetic compound is precipitated calcium carbonate (PCC) that is generally obtained by precipitation following the reaction of carbon dioxide and lime in an aqueous environment or by precipitation of a calcium and carbonate source in water or by precipitation of calcium and carbonate ions, for example CaCl₂ and Na₂CO₃, out of solution. Precipitated calcium carbonate exists in three primary crystalline forms: calcite, aragonite and vaterite, and there are many different polymorphs (crystal habits) for each of these crystalline forms. The three primary crystalline forms of PCC are similar to or the same as those of the natural occurring minerals calcite, aragonite and vaterite.

Furthermore, the present invention refers to the use of at least one linear or cyclic diamine or triamine compound, the linear or cyclic diamine or triamine compound having at least one primary amine group, and where the linear diamine or triamine compound has the following formula (I): 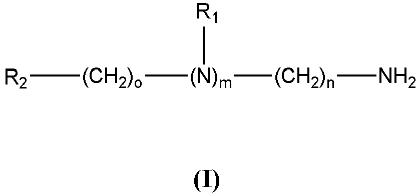 wherein:
m is an integer and is either 0 or 1, n is an integer and is from 2 to 8, o is an integer and is from 0 to 3, R₁ is a C₁ to C₁₂ alkyl group, and in particular a linear C₁₂H₂₅ alkyl group, and R₂ is CH₃ or NH₂, and with the proviso that when m = 0, then o = 0 and R₂ is NH₂;
and where the cyclic diamine or triamine compound has one of the following formulas (II) and (III): 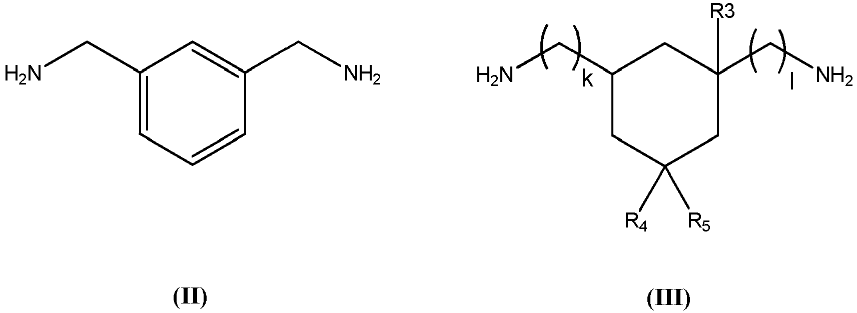 wherein:
k and 1 are the same or different and are either 0 or 1, R₃ is either H or CH₃, and R₄ and R₅ are the same or different and selected from H and CH₃, as a biocidal activity enhancing compound in an aqueous mineral preparation comprising at least one of ground natural calcium carbonate, precipitated calcium carbonate, dolomite, surface-reacted calcium carbonate, clay, talc, TiO₂, kaolin, kaolinitic clay, calcined kaolinitic clay, calcium sulfate, quartz, attapulgite, montmorillonite, diatomaceous earth, finely divided silica, aluminium oxide, aluminium hydroxide, silicates such as aluminium silicate, pumice and sepiolite, or a mixture thereof, and comprising at least one aldehyde-containing and/or aldehyde-releasing and/or phenolic and/or isothiazoline biocide, where the total amount of said biocide(s) in the aqueous preparation is from 90 ppm to 1 350 ppm, calculated relative to the weight of the aqueous phase of said preparation, and the total amount of said linear or cyclic diamine or triamine compound(s) in the aqueous preparation is from 600 to 1 200 ppm, calculated relative to the weight of the aqueous phase of said preparation.

A biocidal activity enhancing compound referred to herein is a compound which is capable of increasing or inducing the biocidal activity of one or more biocides in comparison with a preparation having no such biocidal activity enhancing compound but, e.g. only one or more biocides in an amount such that the total amount of biocides in the aqueous preparation is from 90 to 1 350 ppm, calculated relative to the water in the preparation.

Notably, the biocidal activity enhancing compound may be capable of inducing the biocidal activity of one or more biocides when these biocides are dosed in an amount that is less than their Minimum Inhibition Concentration (MIC), the MIC being defined as the lowest concentration needed to reduce the TVC to the order of 10² cfu/ml.

A first aspect of the present application resides in a process for stabilising an aqueous mineral preparation comprising a step of:
(a) adding at least one aldehyde-containing and/or aldehyde-releasing and/or phenolic and/or isothiazoline biocide to said aqueous mineral preparation;
   characterised in that:
   - said mineral comprises at least one of: a ground natural calcium carbonate, a precipitated calcium carbonate, a dolomite, a surface-reacted calcium carbonate, clay, talc, TiO₂, kaolin, kaolinitic clay, calcined kaolinitic clay, calcium sulfate, quartz, attapulgite, montmorillonite, diatomaceous earth, finely divided silica, aluminium oxide, aluminium hydroxide, silicates such as aluminium silicate, pumice and sepiolite, or a mixture thereof;
   - said process comprises a step (b), which may be simultaneous and/or distinct relative to step (a), of adding at least one to linear or cyclic diamine or triamine compound said aqueous mineral preparation, the linear or cyclic diamine or triamine compound having at least one primary amine group, and where the linear diamine or triamine compound has the following formula (I): 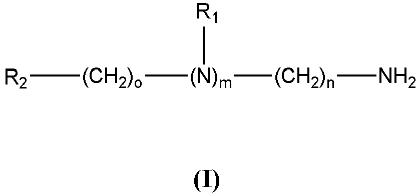 wherein:
      m is an integer and is either 0 or 1, n is an integer and is from 2 to 8, o is an integer and is from 0 to 3, R₁ is a C₁ to C₁₂ alkyl group, and is in particular CH₃, CH₂CH₃ or a linear C₁₂H₂₅ alkyl group, and R₂ is CH₃ or NH₂, and with the proviso that when m = 0, then o = 0 and R₂ is NH₂;
      and where the cyclic diamine or triamine compound has one of the following formulas (II) and (III): 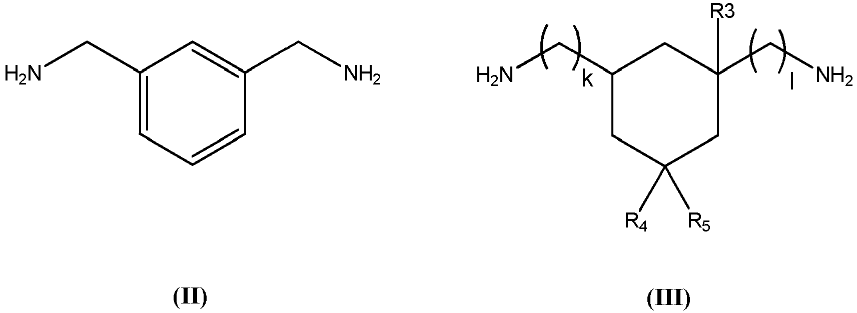 wherein:
      k and 1 are the same or different and are either 0 or 1, R₃ is either H or CH₃, and R₄ and R₅ are the same or different and selected from H and CH₃;
   - said biocide(s) are added to said aqueous preparation in an amount corresponding to from 90 to 1 350 ppm, based on the weight of the aqueous phase of said aqueous preparation; and
   - said linear or cyclic diamine or triamine compound(s) are added to said aqueous preparation in an amount corresponding to from 600 to 1 200 ppm based on the weight of the aqueous phase of said aqueous preparation.

A second aspect of the present invention resides in the use of at least one linear or cyclic diamine or triamine compound, the linear or cyclic diamine or triamine compound having at least one primary amine group, and where the linear diamine or triamine compound has the following formula (I): 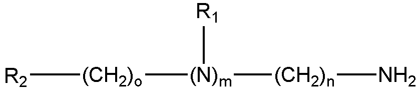 wherein:
m is an integer and is either 0 or 1, n is **(I)** an integer and is from 2 to 8, o is an integer and is from 0 to 3, R₁ is a C₁ to C₁₂ alkyl group, and in particular a linear C₁₂H₂₅ alkyl group, and R₂ is CH₃ or NH₂, and with the proviso that when m = 0, then o = 0 and R₂ is NH₂;
and where the cyclic diamine or triamine compound has one of the following formulas (II) and (III): 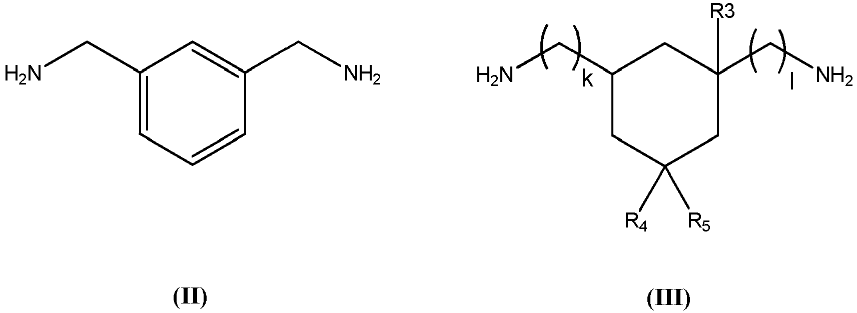 wherein:
k and 1 are the same or different and are either 0 or 1, R₃ is either H or CH₃, and R₄ and R₅ are the same or different and selected from H and CH₃, as a biocidal activity enhancing compound in an aqueous mineral preparation comprising at least one of ground natural calcium carbonate, precipitated calcium carbonate, dolomite, surface-reacted calcium carbonate, clay, talc, TiO₂, kaolin, kaolinitic clay, calcined kaolinitic clay, calcium sulfate, quartz, attapulgite, montmorillonite, diatomaceous earth, finely divided silica, aluminium oxide, aluminium hydroxide, silicates such as aluminium silicate, pumice and sepiolite, or a mixture thereof, and comprising at least one aldehyde-containing and/or aldehyde-releasing and/or phenolic and/or isothiazoline biocide, where the total amount of said biocide(s) in the aqueous preparation is from 90 ppm to 1 350 ppm, calculated relative to the weight of the aqueous phase of said preparation, and the total amount of said linear or cyclic diamine or triamine compound(s) in the aqueous preparation is from 600 to 1 200 ppm, calculated relative to the weight of the aqueous phase of said preparation.

### Biocides

According to a preferred embodiment of the inventive process or use, said aldehyde-containing and/or aldehyde-releasing and/or phenolic and/or isothiazoline biocide(s) are added to the aqueous preparation in a total amount of from 100 ppm to 1 000 ppm, preferably in amount of from 150 ppm to 800 ppm, calculated relative to the water in the preparation.

In one embodiment of the present invention, said biocide(s) are in an undiluted, i.e. concentrated form. In another embodiment, the biocide(s) are diluted to a suitable concentration before being added to the aqueous preparation. In the diluted form, the biocide(s) are preferably dissolved in water, wherein the corresponding diluted composition comprises preferably up to 99 wt.-% of biocide, based on the total weight of the composition. More preferably, the composition in water comprises 50 to 95 wt.-% of biocide and most preferably 60 to 90 wt.-% of biocide, based on the total weight of the composition, whereby the composition may further comprise suitable stabilizers.

The aldehyde-based biocide of the present invention is preferably selected from the group consisting of formaldehyde, acetaldehyde, glyoxal, succinaldehyde, glutaraldehyde, 2-propenal, phthalic dialdehyde and mixtures thereof, and preferably is formaldehyde, glutaraldehyde and mixtures thereof.

In this application glutaraldehyde and glutardialdehyde (GDA) are identical. Both names are widely used in the industry.

Preferred aldehyde-releasing biocides according to the present invention include formaldehyde-releasing biocides, acetaldehyde-releasing biocides, succinaldehyde-releasing biocides, 2-propenal-releasing biocides and mixtures thereof.

According to another embodiment, the aldehyde-releasing compound is selected from the group consisting of benzyl alcoholmono(poly)-hemiformal, ethyleneglycolhemiformal (EGHF), [1,2-Ethanediylbis(oxy)]-bis-methanol, tetrahydro-1,3,4,6-tetrakis(hydroxylmethyl)imidazo[4,5-d]imidazole-2,5(1H,3H)-dione (also commonly referred to as TetraMethylolAcetyleneDiurea TMAD) and mixtures thereof.

Other preferred compounds are those having activated halogen atoms and liberating formaldehyde.

A preferred phenolic biocide is orthophenylphenol (OPP).

A preferred isothiazoline biocide is 2-methyl-4-isothiazoline-3-one (MIT), 5-chloro-2-methyl-2H-isothiazolin-3-one (CIT), 1,2-benzisothiazoline-3-one (BIT), or mixtures thereof.

According to another preferred embodiment of the present invention, the aldehyde-releasing and/or aldehyde-based biocide is used together with biocides selected from the group consisting of 5-chloro-2-methyl-2H-isothiazolin-3-one (CIT), 2-methyl-2H-isothiazolin-3-one (MIT) and mixtures thereof.

The mixtures of biocides which may be used according to the present invention are preferably dissolved in water.

An especially preferred biocide mixture comprises glutaraldehyde, 5-chloro-2-methyl-2H-isothiazolin-3-one (CIT) and 2-methyl-2H-isothiazolin-3-one (MIT).

Another especially preferred biocide mixture comprises ethyleneglycolhemiformal, 5-chloro-2-methyl-2H-isothiazolin-3-one (CIT) and 2-methyl-2H-isothiazolin-3-one (MIT).

### Solids of the aqueous mineral preparation

The water-insoluble solids of the aqueous mineral preparation comprise at least one of: a ground natural calcium carbonate, a precipitated calcium carbonate, a dolomite, a surface-reacted calcium carbonate, clay, talc, TiO₂, kaolin, kaolinitic clay, calcined kaolinitic clay, calcium sulfate, quartz, attapulgite, montmorillonite, diatomaceous earth, finely divided silica, aluminium oxide, aluminium hydroxide, silicates such as aluminium silicate, pumice and sepiolite, or a mixture thereof. In a preferred embodiment of the present invention, said water-insoluble solids of the aqueous mineral preparation comprise at least one ground natural calcium carbonate, a precipitated calcium carbonate, a dolomite, a surface-reacted calcium carbonate.

"Ground natural calcium carbonate" (GNCC) in the meaning of the present invention is a calcium carbonate obtained from natural sources, such as limestone, marble or chalk, and processed through a treatment such as grinding, screening and/or fractionizing by wet and/or dry, for example by a cyclone or classifier.

"Precipitated calcium carbonate" (PCC) in the meaning of the present invention is a synthesized material, generally obtained by precipitation following reaction of carbon dioxide and lime in an aqueous environment or by precipitation of a calcium and carbonate ion source, for example CaCl₂ and Na₂CO₃, in water. Precipitated calcium carbonate exists in three primary crystalline forms: calcite, aragonite and vaterite, and there are many different polymorphs (crystal habits) for each of these crystalline forms. Calcite has a trigonal structure with typical crystal habits such as scalenohedral (S-PCC), rhombohedral (R-PCC), hexagonal prismatic, pinacoidal, colloidal (C-PCC), cubic, and prismatic (P-PCC). Aragonite is an orthorhombic structure with typical crystal habits of twinned hexagonal prismatic crystals, as well as a diverse assortment of thin elongated prismatic, curved bladed, steep pyramidal, chisel shaped crystals, branching tree, and coral or worm-like form.

Said GNCC or PCC may be surface reacted to form a surface-reacted calcium carbonate, which are materials comprising GNCC and/or PCC and an insoluble, at least partially crystalline, non-carbonate calcium salt extending from the surface of at least part of the calcium carbonate. Such surface-reacted products may, for example, be prepared according to WO 00/39222, WO 2004/083316, WO 2005/121257, WO 2009/074492, EP 2 264 108, and EP 2 264 109.

Said GNCC or PCC may additionally be surface treated, for example with fatty acids such as stearic acid and corresponding calcium salts.

In a preferred embodiment of the present invention, said mineral comprises one or more of: ground natural calcium carbonate, precipitated calcium carbonate, dolomite and surface-reacted calcium carbonate, and one or more of: kaolin, kaolinitic clay, calcined kaolinitic clay, talc, calcium sulfate, quartz, attapulgite, montmorillonite, diatomaceous earth, finely divided silica, aluminium oxide, aluminium hydroxide, silicates such as aluminium silicate, pumice and sepiolite, wherein said ground natural calcium carbonate and/or precipitated calcium carbonate and/or dolomite and/or surface-reacted calcium carbonate preferably is present in an amount of greater than or equal to 50 % by weight, more preferably greater than or equal to 60 % by weight, even more preferably greater than or equal to 70 % by weight, even more preferably greater than or equal to 80 % by weight, and most preferably greater than or equal to 90 % by weight, relative to the total weight of the mineral solids.

Clay refers to crystalline small particles of mainly hydrous silicates of aluminium, sometimes with magnesium and/or iron substitution for all or a part of the aluminium. The main groups of clay minerals are: kaolinite, the main constituent of kaolin; halloysite; illite; montmorillonite and vermiculite. The term "kaolinitic clay" used herein refers to a soft white clay that is composed of mainly the mineral kaolinite.

Kaolin is especially used in the paper industry, which uses it to coat and fill papers and boards and improves some of the optical properties of the final product, such as gloss, opacity or brightness. However, kaolin based products include paints, agricultural compositions, fibre glass products, polymers and rubber compositions, ceramic applications, catalyst supports, pharmaceuticals, cosmetics, adhesives, filter aids, and many more.

More preferably, said mineral consists essentially of only ground natural calcium carbonate, precipitated calcium carbonate, dolomite, surface-reacted calcium carbonate or mixture thereof, and most preferably consists only of ground natural calcium carbonate.

Minerals having a positive surface charge at a pH of between 8 and 10 may be particularly advantageous according to the present invention.

In a preferred embodiment, the aqueous mineral preparation has a solids content of from 40 to 82 % by weight, as measured according to the measurement method provided in the Examples section hereafter. More preferably, the solids content is from 50 to 80 % by weight, and even more preferably from 60 to 80 % by weight.

The water-insoluble solid in the preparation may have a particle size distribution as conventionally employed for the material(s) involved in the type of product to be produced. In general, 90 % of the particles will have an esd (equivalent spherical diameter as measured by the well known technique of sedimentation using Sedigraph 5100 series, Micrometrics) of less than 5 µm. Coarse minerals, filler or pigment materials may have a particle esd generally (i.e. at least 90 wt.-%) in the range of 1 to 5 µm. Fine minerals materials may have a particle esd generally less than 2 µm, e.g. 50 to 99 wt.-% less than 2 µm and preferably 60 to 90 wt.-% less than 2 µm. It is preferred that the solid particles in the preparation have a *d*₅₀ value (median esd defined in terms of 50 weight percent of particles being finer than esd, *d*₅₀) of from 0.1 to 5 µm, preferably from 0.2 to 2 µm and most preferably from 0.35 to 1 µm, for example 0.7 µm as measured using a Sedigraph™ 5100 of Micromeritics Instrument Corporation. The method and the instrument are known to the skilled person and are commonly used to determine grain size of fillers. The measurement is carried out in an aqueous solution of 0.1 wt.-% Na₄P₂O₇. The samples are dispersed using a high speed stirrer and supersonics.

For keeping mineral particles in such an aqueous preparation and thus ensuring that the viscosity of the preparation remains substantially the same over time, additives such as dispersing agents, thickeners or anti-settling agents are used. A suitable dispersing agent is preferably made of monomers and/or co-monomers selected from the group consisting of acrylic acid, methacrylic acid, itaconic acid, crotonic acid, fumaric acid, maleic acid, isocrotonic acid, aconitic acid (cis or trans), mesaconic acid, sinapinic acid, undecylenic acid, angelic acid, canellic acid, hydroxyacrylic acid, acrolein, acrylamide, acrylonitrile, dimethylaminoethyl methacrylate, vinylpyrrolidone, vinylcaprolactam, ethylene, propylene, isobutylene, diisobutylene, vinyl acetate, styrene, α-methyl styrene, methyl vinyl ketone, the esters of acrylic and methacrylic acids, such as the acrylates and methacrylates of alkyl, of aryl, of alkylaryl, of arylalkyl, and in particular ethyl acrylate, butyl acrylate, methyl methacrylate, acrylamido methyl propane sulphonic acid, acrylamide and/or methacrylamide, acrylate phosphate of ethylene glycol, methacrylate phosphate of ethylene glycol, acrylate phosphate of propylene glycol acrylate, methacrylate phosphate of propylene glycol, methacrylamido propyl trimethyl ammonium chloride or sulphate, ethyl chloride or ammonium trimethyl methacrylate sulphate, together with their acrylate and acrylamide counterparts, whether or not quaternised, and/or dimethyldiallyl chloride, sodium sulfonate styrene, and mixtures thereof, wherein poly(acrylic acid) and/or poly (methacrylic acid) are preferred as dispersing agent. In addition, dispersing agents based on cellulose or starch can also be used. The skilled man will know how to correctly dose such dispersants to reach an optimal resting and process dispersion viscosity.

### pH of the aqueous mineral preparation

According to a preferred embodiment of the process or the use of the present invention, said aqueous mineral preparation has a pH of between 8 and 10 prior to the addition of any biocide and/or linear or cyclic diamine or triamine compound(s) having at least one primary amine group.

In such a case, the biocides implemented according to the present invention are preferably stable, i.e. not degraded, at a pH of between 8 and 10, at least for a time sufficient to function as a biocide when in combination with the linear or cyclic diamine or triamine compound(s) having at least one primary amine group.

### Linear or cyclic diamine or triamine compound

The linear or cyclic diamine or triamine compound(s) employed in the present invention have at least one primary amine group (NH₂) and are selected from a linear or cyclic diamine or triamine compound, where the linear diamine or triamine compound has the following formula (I): 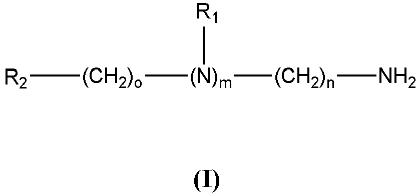 wherein:
m is an integer and is either 0 or 1, n is an integer and is from 2 to 8, o is an integer and is from 0 to 3, R₁ is a C₁ to C₁₂ alkyl group, and is in particular CH₃, CH₂CH₃ or
a linear C₁₂H₂₅ alkyl group, and R₂ is CH₃ or NH₂, and with the proviso that when m = 0, then o = 0 and R₂ is NH₂;
and where the cyclic diamine or triamine compound has one of the following formulas (II) and (III): 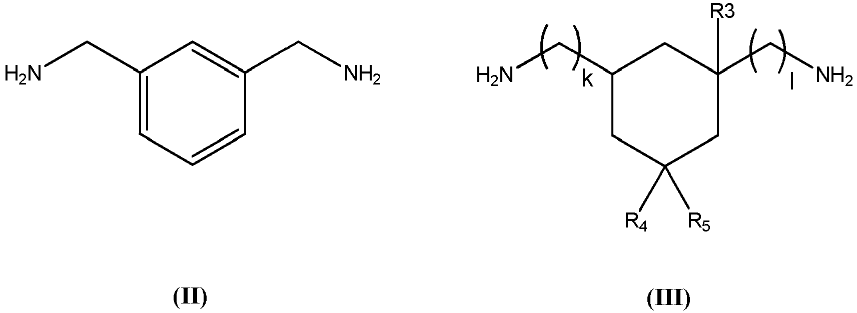 wherein:
k and 1 are the same or different and are either 0 or 1, R₃ is either H or CH₃, and R₄ and R₅ are the same or different and selected from H and CH₃.

It has to be noted that when in formula (I) m is equal 0, then R₁ is also not present in the linear diamine or triamine compound, in other words, group R₂ is directly attached to the (CH₂)ₙ-unit of the (CH₂)ₙ-NH₂ residue.

As regards formula (III), it has to be noted that when any one of k and/or 1 is equal to 0, then the NH₂ group is directly attached to the cyclohexane ring.

In a preferred embodiment of the present invention, said linear or cyclic diamine or triamine compound having at least one primary amine group is selected from the group comprising 1,3-bis(aminomethyl)cyclohexane, 1,3-bis(aminomethyl)benzene, 1,7-diaminoheptane, 1,8-diaminooctane, diethylaminoethylamine, ethylenediamine, N,N-bis-(3-aminopropyl)methylamine, isophorondiamine, and N-(3-aminopropyl)-N-dodecylpropane-1,3-diamine.

### 1,3-Bis(aminomethyl)benzene is represented by formula (II).

1,3-Bis(aminomethyl)cyclohexane is represented by formula (III), wherein k = 1= 1, and R₃ = R₄ = R₅ = H; 1,7-diaminoheptane is represented by formula (I), wherein o = m = 0, R₂ = NH₂, and n = 7; 1,8-diaminooctane is represented by formula (I), wherein o = m = 0, R₂ = NH₂, and n = 8; diethylaminoethylamine is represented by formula (I), wherein m = 1, o = 0; R₁ = CH₂CH₃, R₂ = CH₃, and n = 2; ethylenediamine (also known as "en") is represented by formula (I), wherein o = m = 0, R₂ = NH₂, and n = 2; N,N-bis-(3-aminopropyl)methylamine is represented by formula (I), wherein m = 1, o = 3, R₁ = CH₃, R₂ = NH₂, and n = 3; isophorondiamine is represented by formula (III), wherein k = 0, 1 = 1, and R₃ = R₄ = R₅ = CH₃; and N-(3-aminopropyl)-N-dodecylpropane-1,3-diamine is represented by formula (I), wherein m = 1, n = o = 3, R₁ = linear C₁₂H₂₅ alkyl, and R₂ = NH₂.

In a more preferred embodiment of the present invention said linear or cyclic diamine or triamine compound is selected from the group consisting of 1,3-bis(aminomethyl)cyclohexane, 1,3-bis(aminomethyl)benzene, 1,7-diaminoheptane, 1,8-diaminooctane, diethylaminoethylamine, and ethylenediamine.

The ratios of said biocide(s) to said linear or cyclic diamine or triamine compound(s) having at least one primary amine group may vary over a wide range.

The concentrations of the biocide(s) and the linear or cyclic diamine or triamine compound(s) having at least one primary amine group to be used in the aqueous preparation depend on the nature and the occurrence of the microorganisms to be controlled, the initial microbial load, and on the expected storage time of the aqueous preparations of minerals, fillers or pigments to be protected. The optimum amount to be employed within the defined ranges can be determined by preliminary tests and test series on a laboratory scale and by supplementary operational tests.

In the case where said biocide is an aldehyde-based biocide, such as glutaraldehyde, it is preferred to add said linear or cyclic diamine or triamine compound(s) having at least one primary amine group in an amount such that the weight ratio biocide : linear or cyclic diamine or triamine compound(s) is from 1 : 4 to 1: 1.

In the case where said biocide is a phenolic biocide, such as OPP, it is preferred to add said linear or cyclic diamine or triamine compound(s) having at least one primary amine group in an amount such that the weight ratio biocide : linear or cyclic diamine or triamine compound(s) is from 1 : 4 to 1: 2.

### Order of addition

According to one preferred embodiment of the inventive process said biocide(s) and said linear or cyclic diamine or triamine compound(s) having at least one primary amine group are added separately to the aqueous preparation.

According to another preferred embodiment of the inventive process, said linear or cyclic diamine or triamine compound(s) having at least one primary amine group is added before all or part of said biocide(s). In the alternative, it may especially be preferred according to the inventive process that said biocide(s) are added before all or part of said linear or cyclic diamine or triamine compound(s) having at least one primary amine group.

It is especially preferred to add all of said linear or cyclic diamine or triamine compound(s) having at least one primary amine group before any of said biocide(s).

According to another preferred embodiment of the inventive process, said biocide(s) and said linear or cyclic diamine or triamine compound(s) having at least one primary amine group are added simultaneously. In this embodiment, it is possible that all or part of said biocide(s) are mixed with all or part of said linear or cyclic diamine or triamine compound(s) having at least one primary amine group before addition to the aqueous preparation.

Furthermore, said biocide(s) and/or the linear or cyclic diamine or triamine compound(s) having at least one primary amine group can be added once, e.g. before, during or after the manufacture of the preparation, or several times e.g. in specific time intervals.

### Targeted bacteria

According to the present invention, it is especially preferred that prior to addition of any of said linear or cyclic diamine or triamine compound(s) having at least one primary amine group or said biocide, said aqueous preparation contains bacteria selected from the group consisting of *Thermus sp., Propionibacterium sp., Rhodococcus sp., Panninobacter sp., Caulobacter sp., Brevundimonas sp., Asticcacaulis sp., Sphingomonas sp., Rhizobium sp., Ensifer sp., Bradyrhizobium sp., Tepidimonas sp., Tepidicella sp., Aquabacterium sp., Pelomonas sp., Alcaligenis sp., Achromobacter sp., Ralstonia sp., Limnobacter sp., Massilia sp., Hydrogenophaga sp., Acidovorax sp., Curvibacter sp., Delftia sp., Rhodoferax sp., Alishewanella sp., Stenotrophomonas sp., Dokdonella sp., Methylosinus sp., Hyphomicrobium sp., Methylosulfomonas sp., Methylobacteria sp., Pseudomonas sp.* and mixtures thereof, and more preferably contains bacteria selected from the group consisting of *Pseudomonas putida, Pseudomonas mendocina, Pseudomonas fluorescens, Pseudomonas alcaligenes, Pseudomonas pseudoalcaligenes, Pseudomonas alcaliphila, Pseudomonas entomophila, Pseudomonas syringae, Methylobacterium extorquens, Methylobacterium radiotolerans, Methylobacterium dichloromethanicum, Methylobacterium organophilu, Hyphomicrobium zavarzini* and mixtures thereof.

In one embodiment, the aqueous preparation may further or alternatively contain strains of the above bacteria which are resistant to, tolerant to and/or degrade said biocides in absence of said linear or cyclic diamine or triamine compound(s) having at least one primary amine group.

In the embodiment where the aqueous preparation comprises strains of the above bacteria which are resistant to, tolerant to and/or degrade said biocides in absence of said linear or cyclic diamine or triamine compound(s) having at least one primary amine group, said linear or cyclic diamine or triamine compound(s) having at least one primary amine group in the aqueous preparation is preferably employed in an amount of from 600 to 1 200 ppm, calculated relative to the weight of the aqueous phase of said preparation

In the meaning of the present invention, bacteria which are "resistant" refer to bacteria having the ability to withstand the effects of said biocides when these are dosed in a total amount of from 90 to 1 350 ppm, calculated relative to the amount of water in the preparation. Such resistance evolves naturally via natural selection acting upon random mutation, but it can also be engineered by applying an evolutionary stress on a population.

In the meaning of the present invention, bacteria which are "tolerant" refer to bacteria having the ability to survive in the presence of said biocides without evolving a random mutation.

Bacteria which "degrade" said biocides in the meaning of the present invention correspond to bacteria having the ability to convert said biocides into inactive forms and/or smaller molecules, e.g. by utilizing these substrates as intermediates in their pathways.

Preferably, the inventive process and use provide biocidal activity (stabilization, preservation and/or control of the microbial contamination) of aqueous preparations for a time period of at least 2 days, more preferably for at least 4 days, still more preferably for at least 6 days and most preferably for a minimum of 8 days.

### Applications

According to the present invention, resulting aqueous preparations may be used in many applications, for example, in the field of paper making, paints, detergents and cosmetics.

The following examples may additionally illustrate the invention, but are not meant to restrict the invention to the exemplified embodiments.

### EXAMPLES

### Particle size distribution (mass % particles with a diameter <X) and weight median diameter (d₅₀) of mineral material (i.e. GNCC)

In all of the following examples, the weight median diameter and the particle size distribution characteristics of the mineral material, such as GNCC, are determined via the sedimentation method, i.e. an analysis of sedimentation behavior in a gravimetric field. The measurement is made using a Sedigraph™ 5100 of Micromeritics Instrument Corporation.

The method and the instrument are known to the skilled person and are commonly used to determine grain size of fillers and pigments. The measurement is carried out in an aqueous solution of 0.1 wt% Na₄P₂O₇. The samples are dispersed using a high speed stirrer and supersonics.

### Specific Surface Area (SSA) of a material

The specific surface area is measured via the BET (Brunauer, Emmett, Teller) method according to ISO 9277 using nitrogen, following conditioning of the sample by heating at 250°C for a period of 30 minutes. Prior to such measurements, the sample is filtered, rinsed and dried at 90 to 100°C in an oven for at least 12 hours before being broken down in a mortar with a pestle, and then placed in a weighing balance at 130°C until a constant weight is observed.

### Viscosity measurement

All Brookfield-viscosities are measured with a Brookfield DV-II Viscometer equipped with a LV-3 spindle at a speed of 100 rpm and room temperature (20 ± 3°C).

### Solids content of an aqueous slurry

All mineral preparation solids content (also known as "dry weight") was measured using a Mettler Toledo MJ33 Moisture Analyser.

### Biocide amount and amount of linear or cyclic diamine or triamine compound(s) having at least one primary amine group

All biocide amounts quoted in ppm represent mg values of active content per kilogram of water in the aqueous preparation.

### Total Viable Count (TVC)

All quoted bacterial counts (Total Viable Count (TVC) values are in cfu/ml) in the Tables herebelow are determined after **48 hours** following plate-out and in accordance with the counting method described in "Bestimmung von aeroben mesophilen Keimen", Schweizerisches Lebensmittelbuch, chapter 56, section 7.01, edition of 1985, revised version of 1988.

### Ground Natural Calcium Carbonate Slurry (GNCCS)

The GNCCS used is a slurry of natural ground carbonate (marble). The product is commercialized by Omya, Switzerland, under the trade name Hydrocarb^{®} 90 GU 76.5 %.

The Brookfield-viscosity of the slurry was determined as 350 mPa•s and the pH as 10.

### Biocidal activity in slurry

### Example 1: Ethylene diamine (EDA)

### Aldehyde-based biocide and Isothiazoline biocide mixture (BM1)

Biocide mixture 1 (BM1) is an aqueous solution containing 24 % by weight of GDA (glutaraldehyde /glutardialdehyde) and 1.5 % by weight of a combination of CIT and MIT (5-chloro-2-methyl-2H-isothiazolin-3-one (CIT) and 2-methyl-2H-isothiazolin-3-one (MIT); in a weight ratio CIT : MIT of 3:1), relative to the total solution weight.

BM1 and ethylene diamine (hereafter "EDA") were introduced into 50 g samples of each of the slurries indicated in Table 1 in the listed amounts (quoted in ppm based on the weight of the aqueous phase in the slurry). The samples were then stored at 30°C for 72 hours. References were prepared according to the same protocol but in absence of EDA and in absence of EDA and BM1.

The slurry samples were then inoculated with 1 ml of a mixture of rGDA/IT bacteria culture. Each of the samples was incubated at 30°C for 24 hours. Thereafter, a 1:10 dilution in phosphate buffered saline (PBS) was plated on plate count agar (PCA). These plates were incubated at 30°C and analysed after 48 hours.

The biocide-resistant calcium carbonate slurry culture rGDA/IT is GDA/CIT/MIT-resistant and was obtained from product storage tanks located at white mineral dispersion (WMD) manufacturing plants that used the related biocide to preserve the products. Sequence analysis of the isolated strains revealed that the resistant culture rGDA/IT contained bacteria belonging to the genus *Pseudomonas.* The closest relatives based on the similarity of the 16S rRNA gene sequence were the species *Pseudomonas pseudoalcaligenes, Pseudomonas mendocina* and *Pseudomonas alcaliphila.*

**Table 1**

| Test | Invention (IN) Comparison (CO) | Slurry | Bacteria culture | BM1 (ppm on aqueous phase) | EDA (ppm on aqueous phase) | TVC (cfu/ml) |
|---|---|---|---|---|---|---|
| 1 | CO | GNCCS | rGDA/IT | -- | -- | 1x10⁶ |
| 2 | CO | GNCCS | rGDA/IT | 1350 | -- | 1x10⁶ |
| 3 | CO | GNCCS | rGDA/IT | -- | 1050 | 1x10⁶ |
| 4 | CO | GNCCS | rGDA/IT | 1350 | 450 | 1x10⁶ |
| 5 | IN | GNCCS | rGDA/IT | 1350 | 750 | **1x10²** |
| 6 | IN | GNCCS | rGDA/IT | 675 | 750 | **1x10²** |
| 7 | CO | GNCCS | rGDA/IT | 675 | 450 | 1x10⁴ |

Test 1 shows that there is a growth of the bacterial culture in the slurry in the absence of any one of the biocide BM1 and EDA. Although the biocide BM1 is present in a rather high amount, namely in an amount of 1 350 ppm, Test 2 shows that there is still a significant growth of bacteria in the slurry. This clearly indicates that the biocide BM1 cannot effectively used as a biocide for inhibiting the growth of the rGDA/IT bacteria culture. Test 3 shows that EDA does not provide bacteria inhibiting properties to the slurry. Tests 4 and 7 show the bacterial growth in slurries that were treated with a mixture of the biocide BM1 and EDA. However, it is apparent from these tests, namely a TVC of 1x10⁶ cfu/ml, that the amount of EDA is insufficient to enhance the biocide function of BM1. Finally, Tests 5 and 6 show the bacterial growth in slurries that were treated with a mixture of the biocide BM1 and EDA according to the present invention. From these test results it is evident that an EDA amount of 750 ppm is sufficient to enhance the biocide function of BM1. In summary, the results of the above table confirm that EDA enhances the biocide function of BM1, allowing BM1 to be employed in quantities far below its MIC. Further increasing the amount of EDA allowed the amount of BM1 to be reduced even further relative to its minimum inhibition concentration (MIC).

The above results clearly demonstrate that the biocide enhancer according to the invention provides the necessary enhancement.

Tests 8 and 9 below represent the same slurry as Tests 5 and 6, respectively, but following a second inoculation of 1 ml of a mixture of *Pseudomonas* species.

**Table 2**

| Test | Invention (IN) Comparison (CO) | Slurry | Bacteria culture | BM1 (ppm on aqueous phase) | EDA (ppm on aqueous phase) | TVC (cfu/ml) |
|---|---|---|---|---|---|---|
| 8 | IN | GNCCS | rGDA/IT | 1350 | 750 | **1x10²** |
| 9 | IN | GNCCS | rGDA/IT | 675 | 750 | **1x10²** |

The results of the above table confirm that EDA enhances the biocide function of BM1, allowing BM1 to be employed in quantities below its MIC. The biocide function of EDA is still present after a total of two inoculations of the slurry with the bacteria culture.

Tests 10 and 11 below represent the same slurry as Tests 5 and 6, but following two additional inoculations of each 1 ml of a mixture of *Pseudomonas* species.

**Table 3**

| Test | Invention (IN) Comparison (CO) | Slurry | Bacteria culture | BM1 (ppm on aqueous phase) | EDA (ppm on aqueous phase) | TVC (cfu/ml) |
|---|---|---|---|---|---|---|
| 10 | IN | GNCCS | rGDA/IT | 1350 | 750 | **5.5x10²** |
| 11 | IN | GNCCS | rGDA/IT | 675 | 750 | **1x10²** |

The results of the above table confirm that EDA enhances the biocide function of BM1, allowing BM1 to be employed in quantities below its MIC. The biocide function of EDA is still present after a total of three inoculations of the slurry with the bacteria culture.

### Example 2: Other Diamines

### Aldehyde-based biocide and Isothiazoline biocide mixture (BM1)

Biocide mixture 1 (BM1) is an aqueous solution containing 24 % by weight of (glutaraldehyde /glutardialdehyde) and 1.5 % by weight of a combination of CIT and MIT (in a weight ratio CIT : MIT of 3:1), relative to the total solution weight.

BM1 and a diamine selected from 1,3-bis(aminomethyl)cyclohexane (hereinafter "AMC"), 1,3-bis(aminomethyl)benzene (hereinafter "AMB"), 1,7-diaminoheptane (hereinafter "DAH"), and 1,8-diamionoocatane (hereinafter "DAO") were introduced into 50 g samples of each of the slurries indicated in Table 4 in the listed amounts (quoted in ppm based on the weight of the aqueous phase in the slurry). The samples were then stored at 30°C for 72 hours. References were prepared according to the same protocol but in absence of the respective diamine used and in the absence of the respective diamine and BM1.

The slurry samples were then inoculated with 1 ml of a mixture of rGDA/IT bacteria culture. Each of the samples was incubated at 30°C for 24 hours. Thereafter, a 1:10 dilution in phosphate buffered saline (PBS) was plated on plate count agar (PCA). These plates were incubated at 30°C and analysed after 48 hours.

The biocide-resistant calcium carbonate slurry culture rGDA/IT is GDA/CIT/MIT-resistant and was obtained from product storage tanks located at white mineral dispersion (WMD) manufacturing plants that used the related biocide to preserve the products. Sequence analysis of the isolated strains revealed that the resistant culture rGDA/IT contained bacteria belonging to the genus *Pseudomonas.* The closest relatives based on the similarity of the 16S rRNA gene sequence were the species *Pseudomonas pseudoalcaligenes, Pseudomonas mendocina* and *Pseudomonas alcaliphila.*

**Table 4**

| Test | (IN) / (CO) | Slurry | Bacteria culture | BM1 (ppm) | AMC (ppm) | AMB (ppm) | DAH (ppm) | DAO (ppm) | TVC (cfu/ml) |
|---|---|---|---|---|---|---|---|---|---|
| 12 | CO | GNCCS | rGDA/IT | -- | -- | -- | -- | -- | 1x10⁶ |
| 13 | CO | GNCCS | rGDA/IT | 1350 | -- | -- | -- | -- | 1x10⁶ |
| 14 | CO | GNCCS | rGDA/IT | -- | 750 | -- | -- | -- | 1x10⁶ |
| 15 | CO | GNCCS | rGDA/IT | -- | -- | 750 | -- | -- | 1x10⁶ |
| 16 | CO | GNCCS | rGDA/IT | -- | -- | -- | 750 | -- | 1x10⁶ |
| 17 | CO | GNCCS | rGDA/IT | -- | -- | -- | -- | 750 | 1x10⁶ |
| 18 | CO | GNCCS | rGDA/IT | 1350 | 375 | -- | -- | -- | 1x10⁶ |
| 19 | CO | GNCCS | rGDA/IT | 1350 | -- | 375 | -- | -- | 1x10⁶ |
| 20 | CO | GNCCS | rGDA/IT | 1350 | -- | -- | 375 | -- | 1x10⁶ |
| 21 | CO | GNCCS | rGDA/IT | 1350 | -- | -- | -- | 375 | 1x10⁶ |
| 22 | IN | GNCCS | rGDA/IT | 1350 | 750 | -- | -- | -- | **1x10²** |
| 23 | IN | GNCCS | rGDA/IT | 1350 | -- | 750 | -- | -- | **1x10²** |
| 24 | IN | GNCCS | rGDA/IT | 1350 | -- | -- | 750 | -- | **1x10²** |
| 25 | IN | GNCCS | rGDA/IT | 1350 | -- | -- | -- | 750 | **1x10²** |

Tests 12 to 17 demonstrate the bacterial growth in slurries which have been treated with no biocide (Test 12), only the biocide BM1 (Test 13), and no biocide and only 1,3-bis(aminomethyl)cyclohexane (AMC; Test 14), 1,3-bis(aminomethyl)benzene (AMB; Test 15), 1,7-diaminoheptane (DAH, Test 16), or 1,8-diamionoocatane (DAO; Test 17). From these tests it is apparent that neither one of the aforementioned compounds shows biocide functions with regard to the rGDA/IT bacteria culture in the slurries.

Tests 18 to 21 show the bacteria growth in slurries that were treated with a mixture of the biocide BM1 and EDA. However, it is apparent from these tests, namely a TVC of 1x10⁶ cfu/ml, that the amount of any one of AMC, AMB, DAH, or DAO used (i.e. 375 ppm) is insufficient to enhance the biocide function of BM1.

Finally, Tests 22 to 25 demonstrate the bacterial growth in slurries that were treated with a mixture of the biocide BM1 and any one of AMC, AMB, DAH, or DAO according to the present invention. From these test results it is evident that an AMC, AMB, DAH, or DAO amount of 750 ppm is sufficient to enhance the biocide function of BM1. In summary, the results of the above table confirm that each one of 1,3-bis(aminomethyl)cyclohexane, 1,3-bis(aminomethyl)benzene, 1,7-diaminoheptane, and 1,8-diamionoocatane enhances the biocide function of BM1, allowing BM1 to be employed in quantities far below its minimum inhibition concentration (MIC).

The above results clearly demonstrate that the biocide enhancer according to the invention provides the necessary enhancement.

Tests 25, 26, 27 and 28 below represent the same slurry as Tests 22, 23, 24 and 25, respectively, but following a second inoculation of 1 ml of a mixture of *Pseudomonas* species.

**Table 5**

| Test | (IN) / (CO) | Slurry | Bacteria culture | BM1 (ppm) | AMC (ppm) | AMB (ppm) | DAH (ppm) | DAO (ppm) | TVC (cfu/ml) |
|---|---|---|---|---|---|---|---|---|---|
| 13 | CO | GNCCS | rGDA/IT | 1350 | -- | -- | -- | -- | **1x10⁶** |
| 25 | IN | GNCCS | rGDA/IT | 1350 | 750 | -- | -- | -- | **1x10²** |
| 26 | IN | GNCCS | rGDA/IT | 1350 | -- | 750 | -- | -- | **1x10²** |
| 27 | IN | GNCCS | rGDA/IT | 1350 | -- | -- | 750 | -- | **1x10²** |
| 28 | IN | GNCCS | rGDA/IT | 1350 | -- | -- | -- | 750 | **1x10²** |

The results of the above table confirm that any one of AMC, AMB, DAH, or DAO enhances the biocide function of BM1, allowing BM1 to be employed in quantities below its MIC. The biocide function of any one of AMC, AMB, DAH, and DOH is still present after a total of two inoculations of the slurry with the bacteria culture.

### Example 3: AMC, AMB, DAH, and DAO

### Orthophenylphenol (OPP)

OPP, in the form of an aqueous solution having a concentration of 45 %, and one of **AMC, AMB, DAH, and DAO** were introduced into 50 g samples of each of the slurries indicated in Table 6 in the listed amounts (quoted in ppm active content based on the weight of the aqueous phase in the slurry). References were prepared according to the same protocol but in absence of any one of **AMC, AMB, DAH, and DAO**. A reference was prepared according to the same protocol but in absence of **AMC, AMB, DAH, and DAO** and in absence of any biocide.

The slurry samples were then inoculated with 1 ml of a mixture of *Pseudomonas* species containing predominantly *Pseudomonas putida* and *Pseudomonas stutzeri* that are resistant to OPP. Each of the samples was incubated at 30 °C for 72 hours. Thereafter, a 1:10 dilution in phosphate buffered saline (PBS) was plated on plate count agar (PCA). These plates were incubated at 30 °C and analysed after 24 hours.

The biocide-resistant calcium carbonate slurry culture rOPP is resistant toward OPP and was obtained from product storage tanks located at calcium carbonate slurry manufacturing plants that used the related biocide to preserve the products.

Tests 41, 42, 43, 44 and 45 below represent the same slurry as Tests 36, 37, 38, 39 and 40, respectively, but following a second inoculation of 1 ml of a mixture of *Pseudomonas* species. Tests 46, 47, 48, 49 and 50 below represents the same slurry as Tests 36, 37, 38, 39 and 40, respectively, but following two additional inoculations of each 1 ml of a mixture of *Pseudomonas* species.

**Table 6**

| Test | (IN) / (CO) | Slurry | Bacteria culture | OPP (ppm) | AMC (ppm) | AMB (ppm) | DAH (ppm) | DAO (ppm) | TVC (cfu/ml) |
|---|---|---|---|---|---|---|---|---|---|
| 29 | CO | GNCCS | rOPP | -- | -- | -- | -- | -- | 1x10⁶ |
| 30 | CO | GNCCS | rOPP | 250 | -- | -- | -- | -- | 1x10⁶ |
| 31 | CO | GNCCS | rOPP | -- | 750 | -- | -- | -- | 1x10⁶ |
| 32 | CO | GNCCS | rOPP | -- | -- | 750 | -- | -- | 1x10⁶ |
| 33 | CO | GNCCS | rOPP | -- | -- | -- | 750 | -- | 1x10⁶ |
| 34 | CO | GNCCS | rOPP | -- | -- | -- | -- | 750 | 1x10⁶ |
| 35 | IN | GNCCS | rOPP | 250 | 750 | -- | -- | -- | **1x10²** |
| 36 | IN | GNCCS | rOPP | 250 | -- | 750 | -- | -- | **1x10²** |
| 37 | IN | GNCCS | rOPP | 250 | -- | -- | 750 | -- | **1x10²** |
| 38 | IN | GNCCS | rOPP | 250 | -- | -- | -- | 750 | **1x10²** |

Tests 29 to 34 demonstrate the bacterial growth in slurries which have been treated with no biocide (Test 29), only the biocide BM1 (Test 30), and no biocide and only 1,3-bis(aminomethyl)cyclohexane (AMC; Test 31), 1,3-bis(aminomethyl)benzene (AMB; Test 32), 1,7-diaminoheptane (DAH, Test 33), or 1,8-diamionoocatane (DAO; Test 34). From these tests it is apparent that neither one of the aforementioned compounds shows biocide functions with regard to the rOPP bacteria culture in the slurries.

Tests 35 to 38 demonstrate the bacterial growth in slurries that were treated with a mixture of the biocide BM1 and any one of AMC, AMB, DAH, or DAO according to the present invention. From these test results it is evident that an AMC, AMB, DAH, or DAO amount of 750 ppm is sufficient to enhance the biocide function of BM1. In summary, the results of the above table confirm that each one of 1,3-bis(aminomethyl)cyclohexane, 1,3-bis(aminomethyl)benzene, 1,7-diaminoheptane, and 1,8-diamionoocatane enhances the biocide function of BM1, allowing BM1 to be employed in quantities far below its MIC.

Also, the results of the above table confirm that every one of ethylene diamine (EDA), 1,3-bis(aminomethyl)cyclohexane (AMC), 1,3-bis(aminomethyl)benzene (AMB), 1,7-diaminoheptane (DAH), and 1,8-diamionoocatane (DAO) enhances the biocide function of OPP.

Tests 39, 40, 41, and 42 below represent the same slurry as Tests 35, 36, 37, and 38, respectively, but following a second inoculation of 1 ml of a mixture of *Pseudomonas* species.

**Table 7**

| Test | (IN) / (CO) | Slurry | Bacteria culture | OPP (ppm) | AMC (ppm) | AMB (ppm) | DAH (ppm) | DAO (ppm) | TVC (cfu/ml) |
|---|---|---|---|---|---|---|---|---|---|
| 29 | CO | GNCCS | rOPP | -- | -- | -- | -- | -- | 1x10⁶ |
| 39 | IN | GNCCS | rOPP | 250 | 750 | -- | -- | -- | **1x10²** |
| 40 | IN | GNCCS | rOPP | 250 | -- | 750 | -- | -- | **1x10²** |
| 41 | IN | GNCCS | rOPP | 250 | -- | -- | 750 | -- | **1x10²** |
| 42 | IN | GNCCS | rOPP | 250 | -- | -- | -- | 750 | **1x10²** |

The results of the above table confirm that any one of AMC, AMB, DAH, or DAO enhances the biocide function of BM1, allowing BM1 to be employed in quantities below its MIC. The biocide function of any one of AMC, AMB, DAH, and DOH is still present after a total of two inoculations of the slurry with the bacteria culture rOPP.

Tests 43, 44, 45, and 46 below represent the same slurry as Tests 35, 36, 37, and 38, respectively, but following two additional inoculations of each 1 ml of a mixture of *Pseudomonas* species.

**Table 8**

| Test | (IN) / (CO) | Slurry | Bacteria culture | OPP (ppm) | AMC (ppm) | AMB (ppm) | DAH (ppm) | DAO (ppm) | TVC (cfu/ml) |
|---|---|---|---|---|---|---|---|---|---|
| 29 | CO | GNCCS | rOPP | -- | -- | -- | -- | -- | 1x10⁶ |
| 43 | IN | GNCCS | rOPP | 250 | 750 | -- | -- | -- | **1x10²** |
| 44 | IN | GNCCS | rOPP | 250 | -- | 750 | -- | -- | **1x10²** |
| 45 | IN | GNCCS | rOPP | 250 | -- | -- | 750 | -- | **1x10²** |
| 46 | IN | GNCCS | rOPP | 250 | -- | -- | -- | 750 | **1x10²** |

The results of the above table confirm that any one of AMC, AMB, DAH, or DAO enhances the biocide function of BM1, allowing BM1 to be employed in quantities below its MIC. The biocide function of any one of AMC, AMB, DAH, and DOH is still present after a total of three inoculations of the slurry with the bacteria culture rOPP.

## Claims

1. Process for stabilising an aqueous mineral preparation comprising a step of:
(a) adding at least one aldehyde-containing and/or aldehyde-releasing and/or phenolic and/or isothiazoline biocide to said aqueous mineral preparation;
**characterised in that:**
- said mineral comprises at least one of: a ground natural calcium carbonate, a precipitated calcium carbonate, a dolomite, a surface-reacted calcium carbonate, clay, talc, TiO₂, kaolin, kaolinitic clay, calcined kaolinitic clay, calcium sulfate, quartz, attapulgite, montmorillonite, diatomaceous earth, finely divided silica, aluminium oxide, aluminium hydroxide, silicates, pumice and sepiolite, or a mixture thereof;
- said process comprises a step (b), which may be simultaneous and/or distinct relative to step (a), of adding at least one linear or cyclic diamine or triamine compound to said aqueous mineral preparation, the linear or cyclic diamine or triamine compound having at least one primary amine group, and where the linear diamine or triamine compound has the following formula (I): 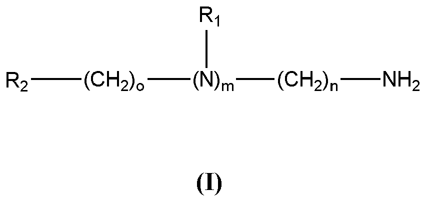 wherein:
m is an integer and is either 0 or 1, n is an integer and is from 2 to 8, o is an integer and is from 0 to 3, R₁ is a C₁ to C₁₂ alkyl group, and R₂ is CH₃ or NH₂, and with the proviso that when m = 0, then o = 0 and R₂ is NH₂;
and where the cyclic diamine or triamine compound has one of the following formulas (II) and (III): 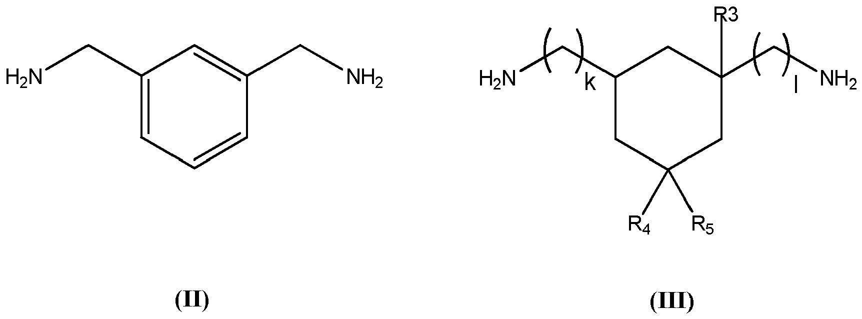 wherein:
k and 1 are the same or different and are either 0 or 1, R₃ is either H or CH₃, and R₄ and R₅ are the same or different and selected from H and CH₃,
- said biocide(s) are added to said aqueous preparation in an amount corresponding to from 90 to 1 350 ppm based on the weight of the aqueous phase of said aqueous preparation; and
- said linear or cyclic diamine or triamine compound(s) are added to said aqueous preparation in an amount corresponding to from 600 to 1 200 ppm, based on the weight of the aqueous phase of said aqueous preparation.

2. Process according to claim 1, **characterized in that** the mineral comprises at least one of: a ground natural calcium carbonate, a precipitated calcium carbonate, a dolomite, a surface-reacted calcium carbonate.

3. Process according to claim 1 or 2, **characterized in that** the mineral comprises aluminium silicate.

4. Process according to any of claims 1 to 3, **characterized in that** R₁ in formula (I) is CH₃, CH₂CH₃ or a linear C₁₂H₂₅ alkyl group.

5. Process according to any one of claims 1 to 4, **characterised in that** said aldehyde-containing and/or aldehyde-releasing and/or phenolic and/or isothiazoline biocide(s) are added to the aqueous preparation in a total amount of from 100 ppm to 1 000 ppm, preferably in amount of from 150 ppm to 800 ppm, calculated relative to the water in the preparation.

6. Process according to any one of claims 1 to 5, **characterised in that** said aldehyde-based biocide is selected from the group consisting of formaldehyde, acetaldehyde, glyoxal, succinaldehyde, glutaraldehyde, 2-propenal, phthalic dialdehyde and mixtures thereof, and preferably is formaldehyde, glutaraldehyde and mixtures thereof, and/or
**characterised in that** said aldehyde-releasing biocides are selected from the group consisting of formaldehyde-releasing biocides, acetaldehyde-releasing biocides, succinaldehyde-releasing biocides, 2-propenal-releasing biocides and mixtures thereof, wherein said aldehyde-releasing biocides preferably are selected from the group consisting of benzyl alcoholmono(poly)-hemiformal, ethyleneglycolhemiformal (EGHF), [1,2-Ethanediylbis(oxy)]-bis-methanol, tetrahydro-1,3,4,6-tetrakis(hydroxylmethyl)imidazo[4,5-d]imidazole-2,5(1H,3H)-dione (also commonly referred to as TetraMethylolAcetyleneDiurea TMAD) and mixtures thereof.

7. Process according to any of claims 1 to 6, **characterised in that** said phenolic biocide is orthophenylphenol (OPP), and/or **characterised in that** said isothiazoline biocide is selected from the group consisting of 2-methyl-4-isothiazoline-3-one (MIT), 5-chloro-2-methyl-2H-isothiazolin-3-one (CIT), 1,2-benzisothiazoline-3-one (BIT), and mixtures thereof.

8. Process according to any of claims 1 to 7, **characterised in that** said aldehyde-releasing and/or aldehyde-based biocide is used together with biocides selected from the group consisting of 5-chloro-2-methyl-2H-isothiazolin-3-one (CIT), 2-methyl-2H-isothiazolin-3-one (MIT) and mixtures thereof.

9. Process according to any one of claims 1 to 5, **characterised in that** said biocides are a combination of glutaraldehyde, 5-chloro-2-methyl-2H-isothiazolin-3-one (CIT) and 2-methyl-2H-isothiazolin-3-one (MIT), or a combination of ethyleneglycol-hemiformal, 5-chloro-2-methyl-2H-isothiazolin-3-one (CIT) and 2-methyl-2H-isothiazolin-3-one (MIT).

10. Process according to any of claims 1 to 9, **characterised in that** said mineral comprises one or more of: ground natural calcium carbonate, precipitated calcium carbonate, dolomite and surface-reacted calcium carbonate, and one or more of: kaolin, kaolinitic clay, calcined kaolinitic clay, talc, calcium sulfate, quartz, attapulgite, montmorillonite, diatomaceous earth, finely divided silica, aluminium oxide, aluminium hydroxide, silicates such as aluminium silicate, pumice and sepiolite, wherein said ground natural calcium carbonate and/or precipitated calcium carbonate and/or dolomite and/or surface-reacted calcium carbonate preferably is present in an amount of greater than or equal to 50 % by weight, more preferably greater than or equal to 60 % by weight, even more preferably greater than or equal to 70 % by weight, even more preferably greater than or equal to 80 % by weight, and most preferably greater than or equal to 90 % by weight, relative to the total weight of the mineral solids.

11. Process according to any of claims 1 to 10, **characterised in that** said mineral consists of only ground natural calcium carbonate, precipitated calcium carbonate, dolomite, surface-reacted calcium carbonate or mixtures thereof, and preferably consists only of ground natural calcium carbonate.

12. Process according to any of claims 1 to 11, **characterised in that** said aqueous mineral preparation has a solids content of from 40 to 82 % by weight, preferably of from 50 to 80 % by weight, and even more preferably of from 60 to 80 % by weight; and/or **characterised in that** said aqueous mineral preparation has a pH of between 8 and 10, prior to the addition of any biocide and/or linear or cyclic diamine or triamine compound having at least one primary amine group.

13. Process according to any of claims 1 to 12, **characterised in that** said linear or cyclic diamine or triamine compound having at least one primary amine group is selected from the group comprising 1,3-bis(aminomethyl)cyclohexane, 1,3-bis(aminomethyl)benzene, 1,7-diaminoheptane, 1,8-diaminooctane, diethylaminoethylamine, ethylenediamine, N,N-bis-(3-aminopropyl)methylamine, isophorondiamine, and N-(3-aminopropyl)-N-dodecylpropane-1,3-diamine, and is preferably selected from the group consisting of 1,3-bis(aminomethyl)cyclohexane, 1,3-bis(aminomethyl)benzene, 1,7-diaminoheptane, 1,8-diaminooctane, diethylaminoethylamine, and ethylenediamine.

14. Process according to any one of claims 1 to 5, **characterised in that** in the case where said biocide is an aldehyde-based biocide, said linear or cyclic diamine or triamine compound having at least one primary amine group is added in an amount such that the weight ratio biocide : linear or cyclic diamine or triamine compound is from 1 : 4 to 1 : 1, or
**characterised in that** in the case where said biocide is a phenolic biocide, said linear or cyclic diamine or triamine compound having at least one primary amine group is added in an amount such that the weight ratio biocide : linear or cyclic diamine or triamine compound is from 1 : 4 to 1 : 2.

15. Process according to any of claims 1 to 14, **characterised in that** said biocide(s) and said linear or cyclic diamine or triamine compound having at least one primary amine group are added separately to the aqueous preparation, wherein all of said linear or cyclic diamine or triamine compound having at least one primary amine group preferably is added before any of said biocide(s).

16. Process according to any of claims 1 to 15, **characterised in that** prior to addition of any of said linear or cyclic diamine or triamine compound having at least one primary amine group or said biocide, said aqueous preparation contains bacteria selected from the group consisting of *Thermus sp., Propionibacterium sp., Rhodococcus sp., Panninobacter sp., Caulobacter sp., Brevundimonas sp., Asticcacaulis sp., Sphingomonas sp., Rhizobium sp., Ensifer sp., Bradyrhizobium sp., Tepidimonas sp., Tepidicella sp., Aquabacterium sp., Pelomonas sp., Alcaligenis sp., Achromobacter sp., Ralstonia sp., Limnobacter sp., Massilia sp., Hydrogenophaga sp., Acidovorax sp., Curvibacter sp., Delftia sp., Rhodoferax sp., Alishewanella sp., Stenotrophomonas sp., Dokdonella sp., Methylosinus sp., Hyphomicrobium sp., Methylosulfomonas sp., Methylobacteria sp., Pseudomonas sp.* and mixtures thereof, and more preferably contains bacteria selected from the group consisting of *Pseudomonas putida, Pseudomonas mendocina, Pseudomonas fluorescens, Pseudomonas alcaligenes, Pseudomonas pseudoalcaligenes, Pseudomonas alcaliphila, Pseudomonas entomophila, Pseudomonas syringae, Methylobacterium extorquens, Methylobacterium radiotolerans, Methylobacterium dichloromethanicum, Methylobacterium organophilu, Hyphomicrobium zavarzini* and mixtures thereof.

17. Process according to claim 16, **characterised in that** all or part of said bacteria are resistant to, tolerant to and/or degrade said biocides in absence of said linear or cyclic diamine or triamine compound(s) having at least one primary amine group.

18. Aqueous preparation obtained by the process of any of claims 1 to 17.

19. Use of at least one linear or cyclic diamine or triamine compound, the linear or cyclic diamine or triamine compound having at least one primary amine group, and where the linear diamine or triamine compound has the following formula (I): 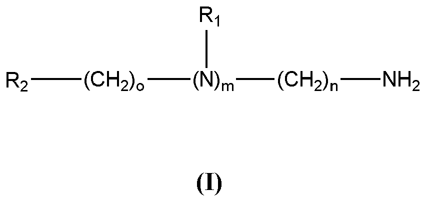 wherein:
m is an integer and is either 0 or 1, n is an integer and is from 2 to 8, o is an integer and is from 0 to 3, R₁ is a C₁ to C₁₂ alkyl group, and R₂ is CH₃ or NH₂, and with the proviso that when m = 0, then o = 0 and R₂ is NH₂;
and where the cyclic diamine or triamine compound has one of the following formulas (II) and (III): 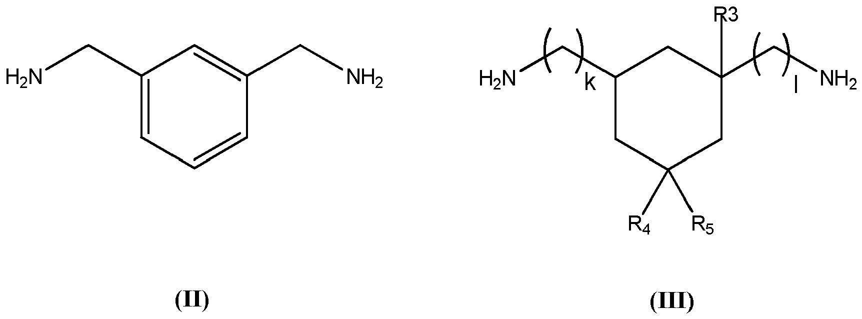
wherein:
k and 1 are the same or different and are either 0 or 1, R₃ is either H or CH₃, and R₄ and R₅ are the same or different and selected from H and CH₃, as a biocidal activity enhancing compound in an aqueous mineral preparation comprising at least one of aqueous ground natural calcium carbonate, precipitated calcium carbonate, dolomite, surface-reacted calcium carbonate, clay, talc, TiO₂, kaolin, kaolinitic clay, calcined kaolinitic clay, calcium sulfate, quartz, attapulgite, montmorillonite, diatomaceous earth, finely divided silica, aluminium oxide, aluminium hydroxide, silicates, pumice and sepiolite, or a mixture thereof, and comprising at least one aldehyde-containing and/or aldehyde-releasing and/or phenolic and/or isothiazoline biocide, where the total amount of said biocide(s) in the aqueous preparation is from 90 ppm to 1 350 ppm, calculated relative to the weight of the aqueous phase of said preparation, and the total amount of said linear or cyclic diamine or triamine compound(s) in the aqueous preparation is from 600 to 1 200 ppm, calculated relative to the weight of the aqueous phase of said preparation.

20. Use according to claim 19, **characterized in that** the mineral comprises at least one of: a ground natural calcium carbonate, a precipitated calcium carbonate, a dolomite, a surface-reacted calcium carbonate.

21. Use according to claim 19 or 20, **characterized in that** the mineral comprises aluminium silicate.

22. Use according to any of claims 19 to 21, **characterized in that** R₁ in formula (I) is a linear C₁₂H₂₅ alkyl group,.

23. Use according to claim 19 or 22, **characterized in that** the linear or cyclic diamine or triamine compound having at least one primary amine group is selected from the group comprising 1,3-bis(aminomethyl)cyclohexane, 1,3-bis(aminomethyl)benzene, 1,7-diaminoheptane, 1,8-diaminooctane, diethylaminoethylamine, ethylenediamine, N,N-bis-(3-aminopropyl)methylamine, isophorondiamine, and N-(3-aminopropyl)-N-dodecylpropane-1,3-diamine, and is preferably selected from the group consisting of 1,3-bis(aminomethyl)cyclohexane, 1,3-bis(aminomethyl)benzene, 1,7-diaminoheptane, 1,8-diaminooctane, diethylaminoethylamine, and ethylenediamine.

24. Use of the aqueous preparation of claim 18 in paper making, paints, detergents or cosmetics.

## Patentansprüche

1. Verfahren zur Stabilisierung einer wässrigen Mineralstoffzubereitung, umfassend einen Schritt:
(a) bei dem wenigstens ein Aldehyd enthaltendes und/oder Aldehyd freisetzendes und/oder Phenol- und/oder Isothiazolin-Biozid zu der wässrigen Zubereitung zugegeben wird;
**dadurch gekennzeichnet, dass:**
- der Mineralstoff wenigstens eines umfasst von: einem vermahlenen, natürlichen Calciumcarbonat, einem gefällten Calciumcarbonat, einem Dolomit, einem Calciumcarbonat, das an der Oberfläche eine Reaktion eingegangen ist, Lehm, Talk, TiO₂, Kaolin, kaolinitischem Lehm, kalziniertem kaolinitischem Lehm, Calciumsulfat, Quarz, Attapulgit, Montmorillonit, Diatomeenerde, fein verteilte Kieselsäure, Aluminiumoxid, Aluminiumhydroxid, Silikate, Bims und Sepiolit, oder eine Mischung davon,
- das Verfahren einen Schritt (b) umfasst, der bezüglich Schritt (a) gleichzeitig und/oder separat sein kann, bei dem wenigstens eine lineare oder zyklische Diamin- oder Triamin-Verbindung zu der wässrigen Mineralstoffzubereitung zugeben wird, wobei die lineare oder zyklische Diamin- oder Triamin-Verbindung wenigstens eine primäre Amingruppe aufweist, und wobei die lineare Diamin- oder Triamin-Verbindung die folgende Formel (I) aufweist: 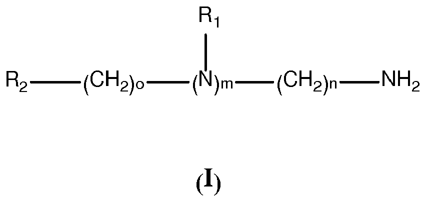 worin:
m eine ganze Zahl und entweder 0 oder 1 ist, n eine ganze Zahl und von 2 bis 8 ist, o eine ganze Zahl und von 0 bis 3 ist, R₁ eine C₁- bis C₁₂-Alkylgruppe ist, und R₂ ist CH₃ oder NH₂, und mit der Massgabe, dass wenn m = 0 ist, dann ist o = 0 und R₂ ist NH₂;
und worin die zyklische Diamin- oder Triamin-Verbindung eine der folgenden Formeln (II) und (III) aufweist: 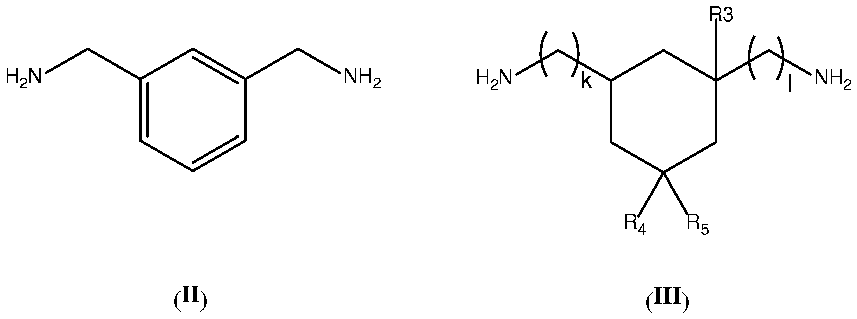 worin:
k und l gleich oder verschieden und entweder 0 oder 1 sind, R₃ ist entweder H oder CH₃, und R₄ und R₅ sind gleich oder verschieden und sind ausgewählt aus H und CH₃,
- das Biozid bzw. die Biozide werden zu der wässrigen Zubereitung in einer Menge zugegeben, die von 90 bis 1 350 ppm entspricht, bezogen auf das Gewicht der wässrigen Phase der wässrigen Zubereitung; und
- die lineare oder zyklische Diamin- oder Triamin-Verbindung bzw. die linearen oder zyklischen Diamin- oder Triamin-Verbindungen werden zu der wässrigen Zubereitung in einer Menge zugegeben, die von 600 bis 1 200 ppm entspricht, bezogen auf das Gewicht der wässrigen Phase der wässrigen Zubereitung.

2. Verfahren nach Anspruch 1, **dadurch gekennzeichnet, dass** der Mineralstoff wenigstens eines umfasst von: einem vermahlenen, natürlichen Calciumcarbonat, einem gefällten Calciumcarbonat, einem Dolomit, einem Calciumcarbonat, das an der Oberfläche eine Reaktion eingegangen ist.

3. Verfahren nach Anspruch 1 oder 2, **dadurch gekennzeichnet, dass** der Mineralstoff Aluminiumsilikat umfasst.

4. Verfahren nach einem der Ansprüche 1 bis 3, **dadurch gekennzeichnet, dass** R₁ in Formel (I) CH₃, CH₂CH₃ oder eine lineare C₁₂H₂₅-Alkylgruppe ist.

5. Verfahren nach einem der Ansprüche 1 bis 4, **dadurch gekennzeichnet, dass** das Aldehyd enthaltende und/oder Aldehyd freisetzende und/oder Phenol- und/oder Isothiazolin-Biozid bzw. die Aldehyd enthaltenden und/oder Aldehyd freisetzenden und/oder Phenol- und/oder Isothiazolin-Biozide zu der wässrigen Zubereitung in einer Gesamtmenge von 100 ppm bis 1 000 ppm zugegeben werden, bevorzugt in einer Menge von 150 ppm bis 800 ppm, berechnet in Bezug auf das Wasser in der Zubereitung.

6. Verfahren nach einem der Ansprüche 1 bis 5, **dadurch gekennzeichnet, dass** das Aldehyd basierende Biozid ausgewählt ist aus der Gruppe bestehend aus Formaldehyd, Acetaldehyd, Glyoxal, Succinaldehyd, Glutaraldehyd, 2-Propenal, Phthalsäuredialdehyd sowie Mischungen davon, und ist bevorzugt Formaldehyd, Glutaraldehyd und Mischungen davon, und/oder **dadurch gekennzeichnet, dass** die Aldehyd freisetzenden Biozide ausgewählt sind aus der Gruppe bestehend aus Formaldehyd freisetzenden Bioziden, Acetaldehyd freisetzenden Bioziden, Succinaldehyd freisetzenden Bioziden, 2-Propenal freisetzenden Bioziden sowie Mischungen davon, wobei die Aldehyd freisetzenden Biozide bevorzugt ausgewählt sind aus der Gruppe bestehend aus Benzylalkoholmono(poly)hemiformal, Ethylenglycolhemiformal (EGHF), [1,2-Ethandiylbis(oxy)]-bis-methanol, tetrahydro-1,3,4,6-tetrakis(hydroxymethyl)imidazo[4,5-d]imidazol-2,5(1H,3H)-dion (auch herkömmlich als TetraMethylolAcetylenDiurea TMAD bezeichnet) sowie Mischungen davon.

7. Verfahren nach einem der Ansprüche 1 bis 6, **dadurch gekennzeichnet, dass** das Phenol-Biozid Orthophenylphenol (OPP) ist, und/oder gekennzeichnet, dass das Isothiazolin-Biozid ausgewählt ist aus der Gruppe bestehend aus 2-Methyl-4-isothiazolin-3-on (MIT), 5-Chlor-2-methyl-2H-isothiazolin-3-on (CIT), 1,2-Benzisothiazolin-3-on (BIT) sowie Mischungen davon.

8. Verfahren nach einem der Ansprüche 1 bis 7, **dadurch gekennzeichnet, dass** das Aldehyd freisetzende und/oder Aldehyd basierte Biozid zusammen verwendet wird mit Bioziden ausgewählt aus der Gruppe bestehend aus 5-Chlor-2-methyl-2H-isothiazolin-3-on (CIT), 2-Methyl-2H-isothiazolin-3-on (MIT), sowie Mischungen davon.

9. Verfahren nach einem der Ansprüche 1 bis 5, **dadurch gekennzeichnet, dass** die Biozide eine Kombination von Glutaraldehyd, 5-Chlor-2-methyl-2H-isothiazolin-3-on (CIT) und 2-Methyl-2H-isothiazolin-3-on (MIT) sind, oder eine Kombination von Ethylenglycolhemiformal, 5-Chlor-2-methyl-2H-isothiazolin-3-on (CIT) und 2-Methyl-2H-isothiazolin-3-on (MIT).

10. Verfahren nach einem der Ansprüche 1 bis 9, **dadurch gekennzeichnet, dass** der Mineralstoff einen oder mehrere umfasst von: vermahlenem, natürlichem Calciumcarbonat, gefälltem Calciumcarbonat, Dolomit und Calciumcarbonat, das an der Oberfläche eine Reaktion eingegangen ist, oder einen oder mehrere von: Kaolin, kaolinitischem Lehm, kalziniertem kaolinitischem Lehm, Talk, Calciumsulfat, Quarz, Attapulgit, Montmorillonit, Diatomeenerde, fein verteilte Kieselsäure, Aluminiumoxid, Aluminiumhydroxid, Silikate, wie Aluminiumsilikat, Bims und Sepiolit, wobei das vermahlene, natürliche Calciumcarbonat und/oder gefällte Calciumcarbonat und/oder Dolomit und/oder Calciumcarbonat, das an der Oberfläche eine Reaktion eingegangen ist, bevorzugt in einer Menge von grösser oder gleich 50 Gew.-% vorhanden ist, bevorzugter grösser oder gleich 60 Gew.-%, noch bevorzugter grösser oder gleich 70 Gew.-%, noch bevorzugter grösser oder gleich 80 Gew.-%, und am meisten bevorzugt grösser oder gleich 90 Gew.-%, bezogen auf das Gesamtgewicht des Mineralfeststoffs.

11. Verfahren nach einem der Ansprüche 1 bis 10, **dadurch gekennzeichnet, dass** der Mineralstoff nur besteht aus vermahlenem, natürlichem Calciumcarbonat, gefälltem Calciumcarbonat, Dolomit, Calciumcarbonat, das an der Oberfläche eine Reaktion eingegangen ist, oder Mischungen davon, und besteht bevorzugt nur aus vermahlenem, natürlichem Calciumcarbonat.

12. Verfahren nach einem der Ansprüche 1 bis 11, **dadurch gekennzeichnet, dass** die wässrige Mineralstoffzubereitung einen Feststoffgehalt von 40 bis 82 Gew.-% aufweist, bevorzugt von 50 bis 80 Gew.-%, und noch bevorzugter von 60 bis 80 Gew.-%; und/oder **dadurch gekennzeichnet, dass** die wässrige Mineralstoffzubereitung, vor der Zugabe jeglichen Biozids und/oder linearen oder zyklischen Diamin- oder Triamin-Verbindung, die wenigstens eine primäre Amingruppe aufweist, einen pH von zwischen 8 und 10 aufweist.

13. Verfahren nach einem der Ansprüche 1 bis 12, **dadurch gekennzeichnet, dass** die lineare oder zyklische Diamin- oder Triamin-Verbindung mit wenigstens einer Amingruppe ausgewählt ist aus der Gruppe umfassend 1,3-Bis(aminomethyl)cyclohexan, 1,3-Bis(aminomethyl)benzol, 1,7-Diaminoheptan, 1,8-Diaminooctan, Diethylaminoethylamin, Ethylendiamin, N,N-Bis-(3-aminopropyl)methylamin, Isophorondiamin und N-(3-aminopropyl)-N-dodecylpropan-1,3-diamin, und ist bevorzugt ausgewählt aus der Gruppe bestehend aus 1,3-Bis(aminomethyl)cyclohexan, 1,3-Bis(aminomethyl)benzol, 1,7-Diaminoheptan, 1,8-Diaminooctan, Diethylaminoethylamin und Ethylendiamin.

14. Verfahren nach einem der Ansprüche 1 bis 5, **dadurch gekennzeichnet, dass** in dem Fall, bei dem das Biozid ein Aldehyd basiertes Biozid ist, die lineare oder zyklische Diamin- oder Triamin-Verbindung mit wenigstens einer primären Amingruppe in einer solchen Menge zugeben wird, dass das Gewichtsverhältnis Biozid : linearer oder zyklischer Diamin- oder Triamin-Verbindung von 1 : 4 bis 1 : 1 beträgt, oder
**dadurch gekennzeichnet, dass** in dem Fall, bei dem das Biozid ein Phenol-Biozid ist, die lineare oder zyklische Diamin- oder Triamin-Verbindung mit wenigstens einer primären Amingruppe in einer solchen Menge zugeben wird, dass das Gewichtsverhältnis Biozid : linearer oder zyklischer Diamin- oder Triamin-Verbindung von 1 : 4 bis 1 : 2 beträgt.

15. Verfahren nach einem der Ansprüche 1 bis 14, **dadurch gekennzeichnet, dass** das Biozid bzw. die Biozide und die lineare oder zyklische Diamin- oder Triamin-Verbindung mit wenigstens einer primären Amingruppe separat zu der wässrigen Zubereitung zugegeben werden, wobei alles an der linearen oder zyklischen Diamin- oder Triamin-Verbindung mit wenigstens einer primären Amingruppe bevorzugt vor jeglichem Biozid bzw. jeglichen Bioziden zugegeben wird.

16. Verfahren nach einem der Ansprüche 1 bis 15, **dadurch gekennzeichnet, dass** vor der Zugabe von jeglicher linearen oder zyklischen Diamin- oder Triamin-Verbindung mit wenigstens einer primären Amingruppe oder dem Biozid, die wässrige Zubereitung Bakterien enthält, ausgewählt aus der Gruppe bestehend aus *Thermars sp., Propionibacteriarm sp., Rhodococcus sp., Panninobacter sp., Caulobacter sp., Brevundimonas sp., Asticcacaulis sp., Sphingomonas sp., Rhizobium sp., Ensifer sp., Bradyrhizobium sp., Tepidimonas sp., Tepidicella sp., Aquabacterium sp., Pelomonas sp., Alcaligenis sp., Achromobacter sp., Ralstonia sp., Limnobacter sp., Massilia sp., Hydrogenophaga sp., Acidovorax sp., Carrvibacter sp., Delftia sp., Rhodoferax sp., Alishewanella sp., Stenotrophomonas sp., Dokdonella sp., Methylosinus sp., Hyphomicrobium sp., Methylosulfomonas sp., Methylobacteria sp., Pseudomonas sp.* und Mischungen davon, und enthält bevorzugter Bakterien, ausgewählt aus der Gruppe bestehend aus *Pseudomonas partida, Pseudomonas mendocina, Pseudomonas fluorescens, Pseudomonas alcaligenes, Pseudomonas pseudoalcaligenes, Pseudomonas alcaliphila, Pseudomonas entomophila, Pseudomonas syringae, Methylobacterium extorquens, Methylobacterium radiotolerans, Methylobacterium dichloromethanicum, Methylobacterium organophilu, Hyphomicrobium zavarzini* und Mischungen davon.

17. Verfahren nach Anspruch 16, **dadurch gekennzeichnet, dass** alle oder ein Teil der Bakterien gegenüber den Bioziden in der Abwesenheit von der bzw. den linearen oder zyklischen Diamin- oder Triamin-Verbindung(en) mit wenigstens einer primären Amingruppe resistent und/oder tolerant sind und/oder abbauen.

18. Wässrige Zubereitung, erhalten durch das Verfahren von einem der Ansprüche 1 bis 17.

19. Verwendung von wenigstens einer linearen oder zyklischen Diamin- oder Triamin-Verbindung, wobei die lineare oder zyklische Diamin- oder Triamin-Verbindung wenigstens eine primäre Amingruppe aufweist, und wobei die lineare Diamin- oder Triamin-Verbindung die folgende Formel (I) aufweist: 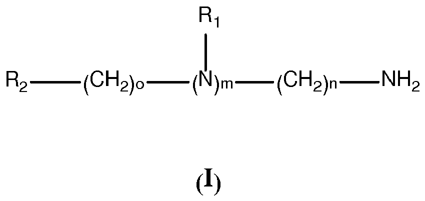 worin:
m eine ganze Zahl und entweder 0 oder 1 ist, n eine ganze Zahl und von 2 bis 8 ist, o eine ganze Zahl und von 0 bis 3 ist, R₁ eine C₁- bis C₁₂-Alkylgruppe ist, und R₂ ist CH₃ oder NH₂, und mit der Massgabe, dass wenn m = 0 ist, dann ist o = 0 und R₂ ist NH₂;
und worin die zyklische Diamin- oder Triamin-Verbindung eine der folgenden Formeln (II) und (III) aufweist: 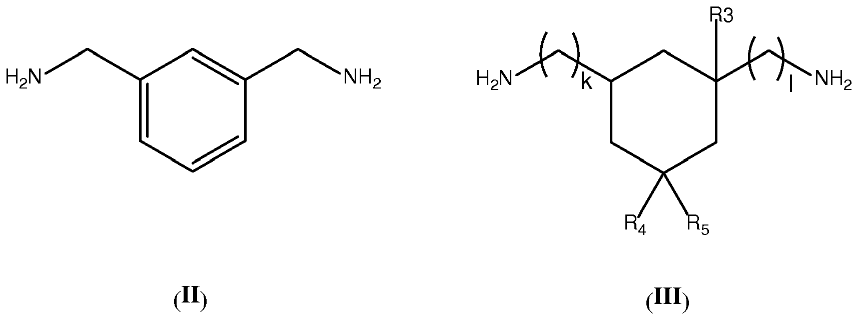 worin:
k und l gleich oder verschieden und entweder 0 oder 1 sind, R₃ ist entweder H oder CH₃, und R₄ und R₅ sind gleich oder verschieden und sind ausgewählt aus H und CH₃, als eine die biologische Aktivität steigernde Verbindung in einer wässrigen Mineralstoffzubereitung, umfassend wenigstens eines aus: wässrigem vermahlenem, natürlichem Calciumcarbonat, gefälltem Calciumcarbonat, Dolomit, Calciumcarbonat, das an der Oberfläche eine Reaktion eingegangen ist, Lehm, Talk, TiO₂, Kaolin, kaolinitischer Lehm, kalziniertem kaolinitischem Lehm, Calciumsulfat, Quarz, Attapulgit, Montmorillonit, Diatomeenerde, fein verteilter Kieselsäure, Aluminiumoxid, Aluminiumhydroxid, Silikate, Bims und Sepiolit, oder eine Mischung davon, und umfasst wenigstens ein Aldehyd enthaltendes und/oder Aldehyd freisetzendes und/oder Phenol- und/oder Isothiazolin-Biozid, wobei die Gesamtmenge des Biozids bzw. der Biozide in der wässrigen Zubereitung von 90 ppm bis 1 350 ppm beträgt, berechnet auf das Gewicht der wässrigen Phase der Zubereitung, und die Gesamtmenge der linearen oder zyklischen Diamin- oder Triamin-Verbindung(en) in der wässrigen Zubereitung beträgt von 600 bis 1 200 ppm, berechnet auf das Gewicht der wässrigen Phase der Zubereitung.

20. Verwendung nach Anspruch 19, **dadurch gekennzeichnet, dass** der Mineralstoff wenigstens eines umfasst von: einem vermahlenen, natürlichen Calciumcarbonat, einem gefällten Calciumcarbonat, einem Dolomit, einem Calciumcarbonat, das an der Oberfläche eine Reaktion eingegangen ist.

21. Verwendung nach Anspruch 19 oder 20, **dadurch gekennzeichnet, dass** der Mineralstoff Aluminiumsilikat umfasst.

22. Verwendung nach einem der Ansprüche 19 bis 21, **dadurch gekennzeichnet, dass** R₁ in Formel (I) eine lineare C₁₂H₂₅-Alkylgruppe ist.

23. Verwendung nach Anspruch 19 oder 22, **dadurch gekennzeichnet, dass** die lineare oder zyklische Diamin- oder Triamin-Verbindung mit wenigstens einer Amingruppe ausgewählt ist aus der Gruppe umfassend 1,3-Bis(aminomethyl)cyclohexan, 1,3-Bis(aminomethyl)benzol, 1,7-Diaminoheptan, 1,8-Diaminooctan, Diethylaminoethylamin, Ethylendiamin, N,N-Bis-(3-aminopropyl)methylamin, Isophorondiamin und N-(3-aminopropyl)-N-dodecylpropan-1,3-diamin, und ist bevorzugt ausgewählt aus der Gruppe bestehend aus 1,3-Bis(aminomethyl)cyclohexan, 1,3-Bis(aminomethyl)benzol, 1,7-Diaminoheptan, 1,8-Diaminooctan, Diethylaminoethylamin und Ethylendiamin.

24. Verwendung der wässrigen Zubereitung von Anspruch 18 bei der Papierherstellung, in Farben, Reinigungsmitteln oder Kosmetika.

## Revendications

1. Procédé de stabilisation d'une préparation minérale aqueuse comprenant une étape consistant à :
(a) ajouter au moins un biocide contenant un aldéhyde et/ou libérant un aldéhyde et/ou un biocide phénolique et/ou à base d'isothiazoline à ladite préparation minérale aqueuse ;
**caractérisé en ce que**
- ledit minéral comprend au moins un parmi : un carbonate de calcium naturel broyé, un carbonate de calcium précipité, une dolomie, un carbonate de calcium ayant réagi en surface, une argile, le talc, TiO₂, le kaolin, l'argile kaolinitique, l'argile kaolinitique calcinée, le sulfate de calcium, le quartz, l'attapulgite , la montmorillonite, une terre de diatomées, une silice finement divisée, l'oxyde d'aluminium, l'hydroxyde d'aluminium, les silicates, la pierre ponce, la sépiolite ou leurs mélanges ;
- ledit procédé comprend une étape (b), qui peut être simultanée et/ou distincte de l'étape (a), consistant à ajouter au moins un composé diamine ou triamine linéaire ou cyclique à ladite préparation minérale aqueuse, le composé diamine ou triamine linéaire ou cyclique ayant au moins un groupe amine primaire, et le composé diamine ou triamine linéaire répondant à la formule suivante (I) : 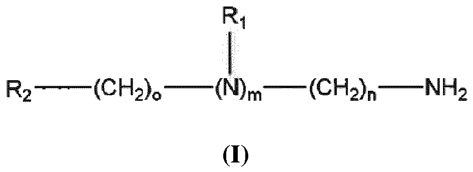 dans laquelle
m est un entier et vaut 0 ou 1, n est un entier et est compris dans la plage allant de 2 à 8, o est un entier et est compris dans la plage allant de 0 à 3, R₁ est un groupe alkyle en C₁ à C₁₂ et R₂ est CH₃ ou NH₂, et à condition que lorsque m = 0, alors o = 0 et R₂ est NH₂ ;
et le composé diamine ou triamine cyclique répondant à une des formules (II) et (III) suivantes : 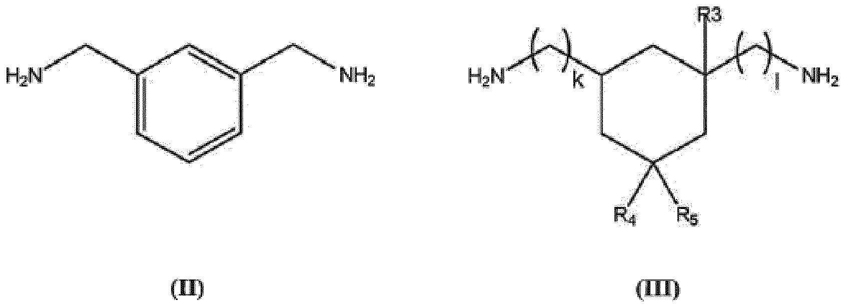 dans lesquelles
k et 1 sont identiques ou différents et valent 0 ou 1, R₃ est H ou CH₃, et R₄ et R₅ sont identiques ou différents et sont choisis parmi H et CH₃,
- ledit ou lesdits biocides sont ajoutés à ladite préparation aqueuse en une quantité correspondant à 90 à 1350 ppm par rapport au poids de la phase aqueuse de ladite préparation aqueuse ; et
- ledit ou lesdits composés diamine ou triamine linéaires ou cycliques sont ajoutés à ladite préparation aqueuse en une quantité correspondant à 600 à 1200 ppm, par rapport au poids de la phase aqueuse de ladite préparation aqueuse.

2. Procédé selon la revendication 1, **caractérisé en ce que** le minéral comprend au moins un de : un carbonate de calcium naturel broyé, un carbonate de calcium précipité, une dolomite, un carbonate de calcium ayant réagi en surface.

3. Procédé selon la revendication 1 ou 2, **caractérisé en ce que** le minéral comprend le silicate d'aluminium.

4. Procédé selon l'une quelconque des revendications 1 à 3, **caractérisé en ce que** R₁ dans la formule (I) est CH₃, CH₂CH₃ ou un groupe alkyle C₁₂H₂₅ linéaire.

5. Procédé selon l'une quelconque des revendications 1 à 4, **caractérisé en ce que** ledit ou lesdits biocides contenant un aldéhyde et/ou libérant un aldéhyde et/ou phénoliques et/ou à base d'isothiazoline sont ajoutés à la préparation aqueuse en une quantité totale de 100 ppm à 1000 ppm, de préférence en une quantité de 150 ppm à 800 ppm, calculée par rapport à l'eau dans la préparation.

6. Procédé selon l'une quelconque des revendications 1 à 5, **caractérisé en ce que** ledit biocide à base d'aldéhyde est choisi dans le groupe constitué par le formaldéhyde, l'acétaldéhyde, le glyoxal, le succinaldéhyde, le glutaraldéhyde, le 2-propénal, le dialdéhyde phtalique et leurs mélanges, et est de préférence le formaldéhyde, le glutaraldéhyde et leurs mélanges, et/ou **caractérisé en ce que** lesdits biocides libérant un aldéhyde sont choisis dans le groupe constitué par les biocides libérant du formaldéhyde, les biocides libérant de l'acétaldéhyde, les biocides libérant du succinaldéhyde, les biocides libérant du 2-propénal et leurs mélanges, lesdits biocides libérant un aldéhyde sont de préférence choisis dans le groupe constitué par le mono(poly)-hémiformal d'alcool benzylique, l'hémiformal d'éthylène glycol (EGHF), le [1,2-éthanediylbis(oxy)]-bis-méthanol, la tétrahydro-1,3,4,6-tétrakis(hydroxylméthyl)imidazo[4,5-d]imidazole-2,5(1H,3H)-dione (également communément appelée TétraMéthylolAcétylèneDiurée : TMAD) et leurs mélanges.

7. Procédé selon l'une quelconque des revendications 1 à 6, **caractérisé en ce que** ledit biocide phénolique est l'orthophénylphénol (OPP), et/ou **caractérisé en ce que** ledit biocide à base d'isothiazoline est choisi dans le groupe constitué par la 2-méthyl-4-isothiazoline-3-one (MIT), la 5-chloro-2-méthyl-2H-isothiazolin-3-one (CIT), la 1,2-benzisothiazoline-3-one (BIT) et leurs mélanges.

8. Procédé selon l'une quelconque des revendications 1 à 7, **caractérisé en ce que** ledit biocide libérant un aldéhyde et/ou à base d'aldéhyde est utilisé avec des biocides choisis dans le groupe constitué par la 5-chloro-2-méthyl-2H-isothiazolin-3-one (CIT), la 2-méthyl-2H-isothiazolin-3-one (MIT) et leurs mélanges.

9. Procédé selon l'une quelconque des revendications 1 à 5, **caractérisé en ce que** lesdits biocides sont une combinaison de glutaraldéhyde, 5-chloro-2-méthyl-2H-isothiazolin-3-one (CIT) et 2-méthyl-2H-isothiazolin-3-one (MIT), ou une combinaison d'hémiformal d'éthylène glycol, 5-chloro-2- méthyl-2H-isothiazoline-3-one (CIT) et 2-méthyl-2H-isothiazoline-3-one (MIT).

10. Procédé selon l'une quelconque des revendications 1 à 9, **caractérisé en ce que** ledit minéral comprend un ou plusieurs parmi : un carbonate de calcium naturel broyé, un carbonate de calcium précipité, une dolomite et un carbonate de calcium ayant réagi en surface, et un ou plusieurs parmi : le kaolin, l'argile kaolinitique, l'argile kaolinitique calcinée, le talc, le sulfate de calcium, le quartz, l'attapulgite, la montmorillonite, une terre de diatomée, une silice finement divisée, l'oxyde d'aluminium, l'hydroxyde d'aluminium, les silicates tels que le silicate d'aluminium, une pierre ponce et la sépiolite, ledit carbonate de calcium naturel broyé et/ou ledit carbonate de calcium précipité et/ou ladite dolomite et/ou ledit carbonate de calcium ayant réagi en surface sont de préférence présents en une quantité supérieure ou égale à 50 % en poids, plus préférablement supérieure ou égale à 60 % en poids, encore plus préférablement supérieure ou égale à 70 % en poids, encore plus préférablement supérieure ou égale à 80 % en poids, et de manière préférée entre toutes supérieure ou égale à 90 % en poids, par rapport au poids total des matières solides minérales.

11. Procédé selon l'une quelconque des revendications 1 à 10, **caractérisé en ce que** ledit minéral se compose uniquement de carbonate de calcium naturel broyé, de carbonate de calcium précipité, de dolomite, de carbonate de calcium ayant réagi en surface ou de leurs mélanges, et de préférence se compose uniquement de carbonate de calcium naturel broyé.

12. Procédé selon l'une quelconque des revendications 1 à 11, **caractérisé en ce que** ladite préparation minérale aqueuse a une teneur en matières solides de 40 à 82 % en poids, de préférence de 50 à 80 % en poids, et encore plus préférablement de 60 à 80 % en poids ; et/ou **caractérisé en ce que** ladite préparation minérale aqueuse a un pH compris entre 8 et 10, avant l'ajout d'un quelconque biocide et/ou d'un composé diamine ou triamine linéaire ou cyclique ayant au moins un groupe amine primaire.

13. Procédé selon l'une quelconque des revendications 1 à 12, **caractérisé en ce que** ledit composé diamine ou triamine linéaire ou cyclique ayant au moins un groupe amine primaire est choisi dans le groupe comprenant le 1,3-bis(aminométhyl)cyclohexane, le 1,3-bis(aminométhyl)benzène, le 1,7-diaminoheptane, le 1,8-diaminooctane, la diéthylaminoéthylamine, l'éthylènediamine, la N,N-bis-(3-aminopropyl)méthylamine, l'isophorondiamine, et la N-(3-aminopropyl)-N-dodécylpropane-1,3-diamine, et est de préférence choisi dans le groupe constitué par le 1,3-bis(aminométhyl)cyclohexane, le 1,3-bis(aminométhyl)benzène, le 1,7-diaminoheptane, le 1,8-diaminooctane, la diéthylaminoéthylamine, et l'éthylènediamine.

14. Procédé selon l'une quelconque des revendications 1 à 5, **caractérisé en ce que** dans le cas où ledit biocide est un biocide à base d'aldéhyde, ledit composé diamine ou triamine linéaire ou cyclique ayant au moins un groupe amine primaire est ajouté en une quantité telle que le rapport en poids de biocide:composé diamine ou triamine linéaire ou cyclique est de 1:4 à 1:1, ou
**caractérisé en ce que** dans le cas où ledit biocide est un biocide phénolique, ledit composé diamine ou triamine linéaire ou cyclique ayant au moins un groupe amine primaire est ajouté en une quantité telle que le rapport en poids de biocide:composé diamine ou triamine linéaire ou cyclique est de 1:4 à 1:2.

15. Procédé selon l'une quelconque des revendications 1 à 14, **caractérisé en ce que** ledit ou lesdits biocides et ledit composé diamine ou triamine linéaire ou cyclique ayant au moins un groupe amine primaire sont ajoutés séparément à la préparation aqueuse, ledit composé diamine ou triamine linéaire ou cyclique ayant au moins un groupe amine primaire étant de préférence ajouté dans sa totalité avant ledit ou lesdits biocides.

16. Procédé selon l'une quelconque des revendications 1 à 15, **caractérisé en ce qu'**avant l'ajout dudit composé diamine ou triamine linéaire ou cyclique ayant au moins un groupe amine primaire ou dudit biocide, ladite préparation aqueuse contient des bactéries choisies dans le groupe constitué par *Thermus sp., Propionibacterium sp., Rhodococcus sp., Panninobacter sp., Caulobacter sp., Brevundimonas sp., Asticcacaulis sp., Sphingomonas sp., Rhizobium sp., Ensifer sp., Bradyrhizobium sp.,Tepidimonas sp., Tepidicella sp., Aquabacterium sp., Pelomonas sp., Alcaligenis sp., Achromobacter sp., Ralstonia sp., Limnobacter sp., Massilia sp., Hydrogenophaga sp., Acidovorax sp., Carrvibacter sp., Delftia sp., Rhocloferax sp., Alishewanella sp., Stenotrophomonas sp., Dokdonella sp., Methylosinus sp., Hyphomicrobium sp., Methylosulfomonas sp., Methylobacteria sp., Pseudomonas sp.* et leurs mélanges, et plus préférablement contient des bactéries choisies dans le groupe constitué par *Pseudomonas putida, Pseudomonas mendocina, Pseudomonas flarorescens, Pseudomonas alcaligenes, Pseudomonas pseudoalcaligenes, Pseudomonas alcaliphila, Pseudomonas entomophila, Pseudomonas syringae, Methylobacterium extorqarens, Methylobacterium radiotolerans, Methylobacteriarm dichloromethanicum, Methylobacterium organophilu, Hyphomicrobium zavarzini* et leurs mélanges.

17. Procédé selon la revendication 16, **caractérisé en ce que** toutes ou une partie desdites bactéries sont résistantes et/ou tolérantes auxdits biocides et/ou les dégradent en l'absence dudit ou desdits composés diamine ou triamine linéaires ou cycliques ayant au moins un groupe amine primaire.

18. Préparation aqueuse obtenue par le procédé selon l'une quelconque des revendications 1 à 17.

19. Utilisation d'au moins un composé diamine ou triamine linéaire ou cyclique, le composé diamine ou triamine linéaire ou cyclique ayant au moins un groupe amine primaire, et le composé diamine ou triamine linéaire ou cyclique répondant à la formule (I) : 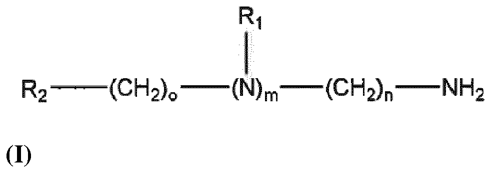 dans laquelle
m est un entier et vaut 0 ou 1, n est un entier et est compris dans la plage allant de 2 à 8, o est un entier et est compris dans la plage allant de 0 à 3, R₁ est un groupe alkyle en C₁ à C₁₂ et R₂ est CH₃ ou NH₂, et à condition que lorsque m = 0, alors o = 0 et R₂ est NH₂ ;
et le composé diamine ou triamine cyclique répondant à une des formules (II) et (III) suivantes : 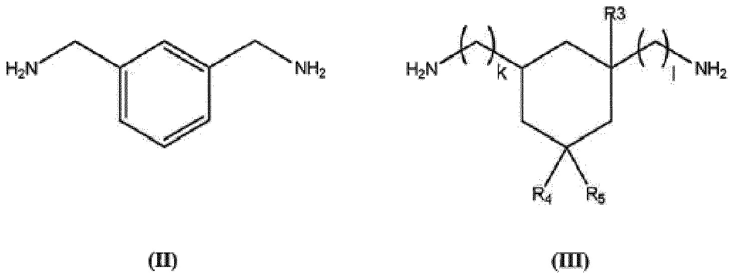 k et 1 sont identiques ou différents et valent 0 ou 1, R₃ est H ou CH₃, et R₄ et R₅ sont identiques ou différents et sont choisis parmi H et CH₃, en tant que composé améliorant l'activité biocide dans une préparation minérale aqueuse comprenant au moins un parmi un carbonate de calcium naturel broyé, un carbonate de calcium précipité, une dolomie, un carbonate de calcium ayant réagi en surface, une argile, le talc, TiO₂, le kaolin, l'argile kaolinitique, l'argile kaolinitique calcinée, le sulfate de calcium, le quartz, l'attapulgite , la montmorillonite, une terre de diatomées, une silice finement divisée, l'oxyde d'aluminium, l'hydroxyde d'aluminium, les silicates, la pierre ponce, la sépiolite ou leurs mélanges, et comprenant au moins un biocide contenant un aldéhyde et/ou libérant un aldéhyde et/ou un biocide phénolique et/ou à base à base d'isothiazoline, où la quantité totale dudit ou desdits biocides dans la préparation aqueuse est de 90 ppm à 1350 ppm, calculée par rapport au poids de la phase aqueuse de ladite préparation, et la quantité totale dudit ou desdites composés diamine ou triamine linéaires ou cycliques dans la préparation aqueuse est de 600 à 1200 ppm, calculée par rapport au poids de la phase aqueuse de ladite préparation.

20. Utilisation selon la revendication 19, **caractérisée en ce que** le minéral comprend au moins un parmi : un carbonate de calcium naturel broyé, un carbonate de calcium précipité, une dolomite, un carbonate de calcium ayant réagi en surface.

21. Utilisation selon la revendication 19 ou 20, **caractérisée en ce que** le minéral comprend le silicate d'aluminium.

22. Utilisation selon l'une quelconque des revendications 19 à 21, **caractérisée en ce que** R₁ dans la formule (I) est un groupe alkyle C₁₂H₂₅ linéaire.

23. Utilisation selon la revendication 19 ou 22, **caractérisée en ce que** ledit composé diamine ou triamine linéaire ou cyclique ayant au moins un groupe amine primaire est choisi dans le groupe comprenant le 1,3-bis(aminométhyl)cyclohexane, le 1,3-bis(aminométhyl)benzène, le 1,7-diaminoheptane, le 1,8-diaminooctane, la diéthylaminoéthylamine, l'éthylènediamine, la N,N-bis-(3-aminopropyl)méthylamine, l'isophorondiamine, et la N-(3-aminopropyl)-N-dodécylpropane-1,3-diamine, et est de préférence choisi dans le groupe constitué par le 1,3-bis(aminométhyl)cyclohexane, le 1,3-bis(aminométhyl)benzène, le 1,7-diaminoheptane, le 1,8-diaminooctane, la diéthylaminoéthylamine, et l'éthylènediamine.

24. Utilisation de la préparation aqueuse selon la revendication 18 dans la fabrication de papier, les peintures, les détergents ou les produits cosmétiques.
